(19) Europäisches Patentamt
European Patent Office
Office européen des brevets

(11) **EP 1 507 475 B1**

(12) **FASCICULE DE BREVET EUROPEEN**

(45) Date de publication et mention de la délivrance du brevet:
**11.07.2012 Bulletin 2012/28**

(21) Numéro de dépôt: **03755190.0**

(22) Date de dépôt: **23.05.2003**

(51) Int Cl.:
*A61B 8/08* (2006.01)     *B06B 1/06* (2006.01)
*G10K 11/00* (2006.01)

(86) Numéro de dépôt international:
**PCT/FR2003/001564**

(87) Numéro de publication internationale:
**WO 2003/099132 (04.12.2003 Gazette 2003/49)**

(54) **PROCEDE ET SONDE POUR EVALUER DE MANIERE NON-INVASIVE UNE DUREE DE PARCOURS OU UNE VITESSE D'ULTRA-SONS LE LONG D'UNE INTERFACE, NOTAMMENT OSSEUSE**

VERFAHREN UND SONDE FÜR DIE NICHTINVASIVE UNTERSUCHUNG EINER FORTBEWEGUNGSZEIT ODER GESCHWINDIGKEIT VON ULTRASCHALL ENTLANG EINER SCHNITTSTELLE, INSBESONDERE EINER KNOCHENSCHNITTSTELLE

METHOD AND PROBE FOR NON-INVASIVE EVALUATION OF A TRAVEL TIME OR SPEED OF ULTRASOUNDS ALONG AN INTERFACE, IN PARTICULAR A BONE INTERFACE

(84) Etats contractants désignés:
**AT BE BG CH CY CZ DE DK EE ES FI FR GB GR HU IE IT LI LU MC NL PT RO SE SI SK TR**

(30) Priorité: **27.05.2002 FR 0206435**

(43) Date de publication de la demande:
**23.02.2005 Bulletin 2005/08**

(73) Titulaire: **CENTRE NATIONAL DE LA RECHERCHE SCIENTIFIQUE (CNRS)**
**75794 Paris Cedex 16 (FR)**

(72) Inventeurs:
• **BOSSY, Emmanuel**
  **F-75010 Paris (FR)**

• **TALMANT, Maryline**
  **F-27200 Vernon (FR)**
• **LAUGIER, Pascal**
  **F-75017 Paris (FR)**

(74) Mandataire: **Grosset-Fournier, Chantal Catherine**
**Grosset-Fournier & Demachy**
**54, rue Saint-Lazare**
**75009 Paris (FR)**

(56) Documents cités:
**EP-A- 0 585 492     WO-A-97/13145**
**SU-A- 1 420 383     US-A- 5 426 979**

**Description**

**Champ de l'invention**

La présente invention concerne un procédé pour évaluer in vivo et de manière non-invasive la durée de propagation entre deux points et/ou la vitesse de propagation d'un signal ultrasonore le long d'une interface de réfraction, en particulier d'une surface osseuse, ainsi qu'une sonde et un appareil d'auscultation osseuse pour sa mise en oeuvre.
Par signal, on entend d'une manière générale toute particularité identifiable du ou des modes vibratoires induits le long de l'interface par le signal d'excitation. On entend aussi, plus particulièrement le premier signal parvenant aux récepteurs, suite à l'application du signal d'excitation.

**Contexte, Etat de l'art**

Il est connu dans l'art que la propagation des ultrasons dans un matériau dépend des propriétés mécaniques et de la géométrie de ce matériau. Ainsi de nombreux dispositifs utilisent la propagation des ultrasons dans les os, en particulier la mesure de vitesse de propagation, dans le but de dépister les modifications subies par l'os au cours de l'ostéoporose ou d'autres pathologies osseuses connues pour affecter la résistance ou l'intégrité osseuse. La majorité des dispositifs commerciaux utilisent une technique de propagation dite transverse dans laquelle deux transducteurs ultrasonores sont disposés de part et d'autre de l'os à étudier; pour étudier la propagation des ultrasons à travers l'os [Brevet Laugier/Berger WO 9526160]. La disposition particulière des capteurs inhérente à une telle technique la limite à l'étude de sites osseux périphériques tels que le talon, les phalanges ou le radius distal, qui sont accessibles à la mesure sur leurs deux faces opposées. Des techniques permettant l'étude spécifique de l'os cortical, os qui ne présente qu'une seule face accessible, applicables sur des os longs tels que le tibia ou le corps du radius ont été proposées depuis de nombreuses années, mettant en jeu la propagation des ultrasons le long de la surface de l'os [G. Lowet and G. Van der Perre, "Ultrasound velocity measurement in long bones: Measurement method and simulation of ultrasound wave propagation," J Biomech 29(10), 1255-1262 (1996), I. Siegel, G. T. Anast and T. Melds, "The determination of fracture healing by measurement of sound velocity across the fracture site," Surgery, Gynecol, Obstetrics, 327-332 (1958).]. La présente invention concerne en particulier ces phénomènes de propagation le long de la surface de l'os.
Pour l'évaluation de l'os in vivo, il est connu que la vitesse de propagation, qui dépend des propriétés mécaniques et géométriques de l'os, peut permettre un diagnostic de l'état de l'os [C. Njeh, D. Hans, T. Fuerst, C. C. Glüer and H. K. Genant (1999). Quantitative ultrasound assessment of osteoporosis and bone status. London, Martin Dunitz.]. Il a ainsi été montré que la mesure de la vitesse d'une onde ultrasonore (de fréquence de l'ordre de 1 MHz) se propageant le long de la surface de l'os cortical du radius permet de discriminer une population saine d'une population ostéoporotique ayant subi une fracture de la hanche [M. Weiss, A. Ben-Shlomo, P. Hagag and S. Ish-Shalom, "Discrimination of proximal hip fracture by quantitative ultrasound measurement at the radius", Osteoporosis Int. 11, 411-416 (2000)]. L'effet des tissus mous dans ce genre de techniques est important, car en général l'épaisseur des tissus mous et leurs propriétés ultrasonores ne sont pas constantes le long de l'os, ce qui peut conduire à des estimations biaisées des paramètres mesurés. Mais la mesure de vitesse se base notamment sur une mesure du temps de parcours de l'onde ultrasonore entre un émetteur et un récepteur placés à une certaine distance l'un de l'autre sur la peau. Or ce temps de parcours est affecté par le trajet de l'onde dans les tissus mous situés d'une part entre l'émetteur et l'os, d'autre part entre l'os et le récepteur.

**Antériorités**

*Brevets négligeant l'effet des tissus mous.*

Les brevets SU1175435, SU1308319, et SU1342479 décrivent des méthodes de mesure de la vitesse de propagation le long de la surface de l'os, de la première onde atteignant successivement plusieurs récepteurs, en utilisant les temps d'arrivée aux récepteurs, et connaissant les positions des récepteurs.
SU1175435 et SU1308319: utilisent un réseau linéaire de transducteurs, avec un élément émetteur et une succession de plusieurs éléments récepteurs également répartis le long d'une barrette. L'épaisseur des tissus mous est considérée constante, ou négligeable.
SU1342479 utilise un émetteur et deux récepteurs, et calcule la vitesse de groupe connaissant la distance entre les deux récepteurs. L'épaisseur des tissus mous est supposée constante.

*Brevets tenant compte de l'effet des tissus mous.*

Les brevets US5143072, US5143069, WO9713145et WO 9945348décrivent des méthodes de mesure de la vitesse de propagation le long de la surface de l'os ne prenant en compte que la première onde arrivant aux récepteurs, en utilisant les temps d'arrivée aux récepteurs, et connaissant les positions des récepteurs. Ces méthodes utilisent des dispositifs à un ou deux émetteurs et un ou deux récepteurs.

US5143072enseigne de calculer la vitesse à partir des différences de temps d'arrivée de la première onde arrivant sur 2 récepteurs, connaissant la distance entre les récepteurs. L'émission ne se produit que quand la surface de la sonde est bien parallèle à la surface de l'os, afin d'avoir une épaisseur constante de tissus mous. Le système teste le parallélisme sonde/os par des tirs en réflexion sur la surface de l'os. A cet effet, le dispositif utilise en mode échographique les éléments récepteurs utilisés pour la détection de l'onde se propageant dans l'os.

US5143069: enseigne d'utiliser un émetteur et deux récepteurs et de calculer une fonction de corrélation des premiers signaux détectés au niveau des deux récepteurs du dispositif, qui conduit à séparer l'énergie propagée dans les tissus mous et l'énergie propagée dans l'os, ainsi qu'à la mesure d'une vitesse dans l'os.

WO9713145: enseigne de corriger l'effet des tissus mous par mesure de la vitesse dans les tissus mous et de l'épaisseur des tissus mous dans la zone de réception, en utilisant des trajets d'ondes en mode échographique n'impliquant pas l'os d'une part, et des trajets d'ondes le long de la surface de l'os d'autre part.

WO9945348: enseigne de prendre en compte l'effet des tissus en résolvant un système d'équations obtenu à partir de quatre chemins de propagation différents. La solution de ce système d'équations donne la vitesse de propagation dans les tissus mous, l'épaisseur des tissus mous et la vitesse de propagation dans l'os.

Une méthode de deux tirs à partir de points opposés et dans des sens opposés le long de la surface d'étude est connue des géophysiciens qui l'utilisent afin de déterminer la profondeur et l'inclinaison d'une interface souterraine, ainsi que la vitesse le long de cette interface, connaissant la vitesse de propagation dans le milieu situé au-dessus, en mesurant le temps de propagation entre émetteur et récepteur [J.L Mari, Géophysique de gisement et de génie civil, Editions Technip, Publications de l'institut Français du Pétrole). Ces mesures sont réalisées sur des distances de plusieurs mètres à quelques centaines de mètres, avec des ondes basse fréquence de l'ordre du Hz à quelques dizaines de Hz , pour des interfaces situées à grande profondeur.

Le brevet SU1420383 enseigne de mesurer la vitesse à partir de deux groupes de transducteurs ultrasonores par des tirs dans deux sens opposés le long de la surface de l'os, les deux groupes de transducteurs pouvant être tour à tour émetteurs et récepteurs. Lors des tirs dans les deux sens, les zones de réception sont distinctes lors de chacun des tirs. La variation des tissus mous ne peut-être qu'imparfaitement corrigée car elle peut être différente sous les deux zones de réception distinctes.

Tous les dispositifs connus permettant la correction d'effets des tissus mous font intervenir la mesure de paramètres tels que l'épaisseur de tissus mous et la vitesse des ultrasons dans les tissus mous. La complexité de telles méthodes et la mesure simultanée de plusieurs grandeurs sont à l'origine du cumul des erreurs inhérentes à chaque étape de mesure, et limitent la précision et l'exactitude de la mesure de la vitesse du premier signal.

## L'invention

Le but de l'invention est ainsi de rendre plus simple et plus efficace la mesure de la durée de propagation entre deux points et/ou de la vitesse de propagation d'un signal le long d'une interface de réfraction, en particulier la surface d'un os. La présente invention a également pour but de proposer une sonde et un appareil pour une auscultation osseuse plus simple et plus efficace.

Suivant un premier aspect de l'invention, le procédé pour évaluer de manière non-invasive une durée de parcours et/ou une vitesse de propagation de signaux ultrasonores entre deux points le long d'une interface dans un corps, en particulier le long d'une surface osseuse, dans lequel, au moyen d'émetteurs et de récepteurs placés à la surface accessible du corps le long de l'interface à ausculter, on détermine pour chacun des deux sens de propagation opposés le long de l'interface l'écart de temps de parcours jusqu'à au moins deux récepteurs espacés le long de la direction de propagation étudiée, est caractérisé en ce qu'on utilise des émetteurs placés de part et d'autre des au moins deux récepteurs, de façon que les mêmes récepteurs reçoivent les signaux pour les deux sens de propagation.

On appelle « écart de temps de parcours jusqu'à deux récepteurs » la différence entre le temps de parcours de la particularité de signal jusqu'à l'un des récepteurs et le temps de parcours de la même particularité ou d'une particularité identique mais résultant d'un tir différent jusqu'à l'autre récepteur.

Pour réaliser une mesure de la vitesse d'une particularité de signal et en particulier d'une particularité de signal choisie comme indicative de l'arrivée du premier signal aux récepteurs, mesure simple, rapide et indépendante de l'effet des tissus mous, on met en oeuvre deux séries de brefs signaux - ou tirs - successifs, à partir de deux zones d'émission situées de part et d'autre d'un groupe de récepteurs. Chaque tir est typiquement un bref train d'alternances ultrasonores. Lors de la première série de tirs, la mesure de la différence entre les temps mis par la particularité de signal pour parvenir aux deux récepteurs, respectivement, donne la vitesse apparente du signal dans un sens. La même méthode appliquée à la deuxième série de tirs donne la vitesse apparente de la particularité de signal dans le sens opposé au précédent. Généralement, le groupe de récepteurs fait un angle avec la surface de l'os située sous lui car les tissus mous ont en général une épaisseur qui n'est pas constante, et les deux écarts de temps obtenus chacun pour l'un des sens de parcours sont donc différents. On obtient la vitesse correcte par une combinaison simple des deux vitesses obtenues. On s'affranchit ainsi de la nécessité de positionner la surface de la sonde parallèlement à la surface de l'os, comme décrit dans les brevets US5143072, US5426979et WO0024307. On s'affranchit aussi de mesures additionnelles de l'épaisseur des tissus mous et de la vitesse de propagation dans les tissus mous (comme décrit dans les brevets WO9713145 et WO9945348) qui ne peuvent que limiter la précision par cumul d'erreur.

Ainsi, on obtient déjà une bonne approximation de la vitesse de propagation du signal le long de l'interface d'après la formule $V_{osa0} = 2 \dfrac{\Delta r}{\Delta t^+ + \Delta t^-}$ où :

$V_{osa0}$ est la valeur approchée de la vitesse de propagation du signal,

$\Delta r$ est la distance entre les deux récepteurs,

$\Delta t^+$ et $\Delta t^-$ sont les écarts de temps de parcours d'un signal jusqu'aux deux récepteurs, dans l'un et respectivement dans l'autre sens de propagation. Cette formule est donc indépendante de la connaissance de l'épaisseur du milieu intercalaire (tissus mous), de l'angle d'inclinaison de la sonde par rapport à l'interface, et de la vitesse de transmission du signal par les tissus mous.

Cette formule revient simplement à prendre

$$\Delta t_0 = \frac{\Delta t^+ + \Delta t^-}{2}$$

Il est donc bien confirmé que le procédé donne le temps de parcours du signal le long de l'interface entre deux points, et permet donc de dater le passage d'une particularité d'un signal en chaque point (de l'interface) d'où le signal est réfracté vers l'un respectif des récepteurs, dans un repère de temps ayant par exemple comme origine l'instant de tir.

Si l'on souhaite une meilleure précision, on peut effectuer au moins une itération comprenant les trois calculs suivants, dans lesquels k représente le rang de l'itération:

a) évaluation $\beta_k$ de l'angle $\beta$ selon lequel le signal traverse le milieu intercalaire situé entre l'interface et les récepteurs, par la formule :

$$\mathrm{Sin}\ \beta_k \cong \frac{V_{tme}}{V_{osa(k-1)}}$$

dans laquelle :

$V_{tme} =$ vitesse supposée du signal dans le milieu intercalaire ;

$V_{osa(k-1)} =$ dernière vitesse estimée de la propagation le long de l'interface

b) nouvelle évaluation $\alpha_k$ de l'angle d'inclinaison ($\alpha$) de l'interface par rapport à la rangée de récepteurs :

$$\tan(\alpha_k) = 2 \left[ \frac{\Delta t^+ - \Delta t^-}{\Delta t^+ + \Delta t^-} \right]\ \tan(\beta_k)$$

c) détermination de la nouvelle valeur approchée de sa vitesse de propagation.

$$V_{osak} = V_{osa0}\ \mathrm{Cos}\ (\alpha_k)$$

Donc une approximation améliorée du temps de parcours est :

$$\Delta t_k = \Delta t_0 / \cos {}'(\alpha_k)$$

Bien-entendu, cette procédure itérative englobe expressément tout calcul itératif identique mais présenté différemment. En particulier, les trois formules a), b) et c) peuvent se combiner en une seule.

En pratique, on se contente souvent de n'effectuer qu'une seule de ces itérations car la précision ainsi obtenue est déjà amplement suffisante pour des applications du type auscultation osseuse.

On peut également utiliser une rangée de récepteurs en nombre supérieur à deux, déterminer pour chaque sens de propagation la pente $1/V^+$ et respectivement $1/V^-$ d'une droite représentative des instants de réception d'un signal en fonction de la position du récepteur le long de la rangée, et déterminer la vitesse approchée du signal dans l'os d'après la formule

$$V_{oso0} = \frac{2}{\dfrac{1}{V^+} + \dfrac{1}{V^-}}$$

Cette évaluation peut être affinée par le même procédé itératif que décrit précédemment.

On peut utiliser ainsi un grand nombre de récepteurs disposés aussi près que techniquement possible l'un de l'autre le long de la direction de propagation étudiée et obtenir une mesure de la vitesse de propagation qui est quasiment indépendante des aléas de mesure qui peuvent affecter la mesure par l'un des deux récepteurs lorsque l'on n'utilise que deux récepteurs.

Pour déterminer l'écart de temps de parcours du signal jusqu'à un premier et jusqu'à un deuxième récepteur, il est préféré de faire la différence entre le temps de parcours d'au moins un premier signal jusqu'au premier récepteur et le temps de parcours d'au moins un deuxième signal jusqu'au deuxième récepteur. Ainsi, on active à chaque fois un seul récepteur et on n'a donc pas besoin de deux ensembles capables de traiter de façon quasi-simultanée les signaux reçus par deux récepteurs.

Le terme « signal » englobe toute particularité identifiable d'un signal. Chaque tir génère le long de l'os un ensemble de modes vibratoires, de sorte que, suite à un tir, chaque récepteur reçoit une combinaison d'ondes assez complexe. En appliquant le procédé selon l'invention, à chaque point du signal complexe, on peut déterminer pour chaque récepteur l'instant où chaque point du signal quitte l'interface pour être réfracté vers ce récepteur, à compter par exemple de l'instant du tir, et donc indépendamment de l'inclinaison de la rangée de récepteurs, de l'épaisseur du milieu intercalaire, et des particularités physiques du milieu intercalaire.

Les signaux d'excitation sont de préférence des tirs séparés par des interruptions ou silences, ce qui permet de mesurer, en tant que vitesse de propagation du premier signal, la vitesse d'un groupe d'alternances facile à repérer puisqu'il est précédé par une interruption et suivi par une interruption.

Il est avantageux d'utiliser des récepteurs qui sont allongés transversalement à la direction de propagation étudiée. Ils peuvent être ainsi relativement étroits, dans la direction de propagation, et ainsi distinguer clairement la présence ou l'absence de signal reçu en un point déterminé de la direction de propagation, tout en présentant grâce à leur allongement une surface sensible dont l'aire est relativement grande.

Il est préféré d'utiliser un signal dont la demi-longueur d'onde dans l'eau correspond approximativement à la dimension de chaque récepteur dans la direction de propagation étudiée. Lorsque le signal est une onde ultrasonore, on peut ainsi utiliser un signal dont la fréquence est de l'ordre du MHz avec des récepteurs dont la largeur est d'environ 0,75 mm suivant la direction de propagation.

Il est avantageux de produire le signal à l'aide d'au moins deux émetteurs qui se succèdent suivant la direction de propagation étudiée, et que l'on synchronise pour qu'ils émettent en concordance spatiale de phase. Ainsi, les deux signaux superposés ont une amplitude qui est égale à la somme de leurs amplitudes individuelles respectives le long de l'interface. La détection du signal reçu par les récepteurs est alors facilitée.

Suivant un second aspect de l'invention, la sonde à ultrasons pour l'auscultation osseuse avec mise en oeuvre d'un procédé selon le premier aspect, cette sonde comprenant des émetteurs et des récepteurs d'ultrasons répartis le long d'une paroi de contact avec la peau du patient, est caractérisée en ce que les émetteurs sont placés dans deux zones d'émission situées de part et d'autre d'une zone de réception ou se trouvent les récepteurs.

De préférence, il est prévu une barrière absorbante pour les ultrasons entre la zone de réception et chacune des zones d'émission. Cette barrière empêche la transmission directe du signal le long de la surface de contact entre la sonde et la peau du patient ou à travers la sonde elle-même.

Suivant un troisième aspect de l'invention, l'appareil pour évaluer in-vivo et de manière non-invasive les propriétés mécaniques d'un os, est caractérisé en ce qu'il comprend :

- une sonde selon le deuxième aspect de l'invention,
- des moyens de commande d'émission pour commander de manière décalée dans le temps au moins un signal ultrasonore à partir de chaque zone d'émission, dans un sens de propagation respectif vers la zone de réception,
- des moyens pour déterminer pour au moins deux récepteurs et pour chaque sens de propagation un écart de temps de parcours jusqu'à ces deux récepteurs déterminés, et
- des moyens pour calculer en fonction des deux écarts de temps de parcours une durée de propagation approximative, ou une vitesse de propagation approximative correspondante.

D'autres particularités et avantages de l'invention résulteront encore de la description ci-après, relative à un exemple non-limitatif.

Aux dessins annexés :

- la figure 1 est une vue schématique de la sonde en coupe dans le plan des transducteurs ;
- la figure 2 est une vue partielle et schématique de la sonde de la figure 1, à échelle agrandie, en coupe dans un plan longitudinal perpendiculaire au plan des transducteurs;
- la figure 3 est un schéma-bloc de l'appareil selon l'invention ;
- la figure 4 est un schéma de principe d'un cycle de mesure ;
- la figure 5 est un diagramme des trajets de propagation explicatif d'un premier mode d'évaluation de la vitesse selon l'invention ;
- la figure 6 est un diagramme explicatif du procédé d'évaluation suivant une variante ;
- la figure 7 est un schéma illustrant l'étape de calibrage ;
- la figure 8 est un diagramme espace-temps relatif à l'évaluation de la vitesse de propagation après calibrage ;
- la figure 9 est un diagramme illustrant la précision du procédé selon l'invention en fonction de l'angle entre la rangée de transducteurs et l'interface à ausculter ;
- la figure 10 représente deux graphiques montrant la vitesse apparente normalisée pour la transmission directe et la transmission inverse, et la vitesse normalisée corrigée en fonction de l'angle d'inclinaison $\alpha$, dans les cas a) d'un échantillon de plexiglas® plongé dans l'eau et b) d'un échantillon d'aluminium plongé dans l'eau ; et
- la Figure 11 est un graphique montrant la vitesse de l'onde de volume en compression dans le plexiglas® en fonction de la température.

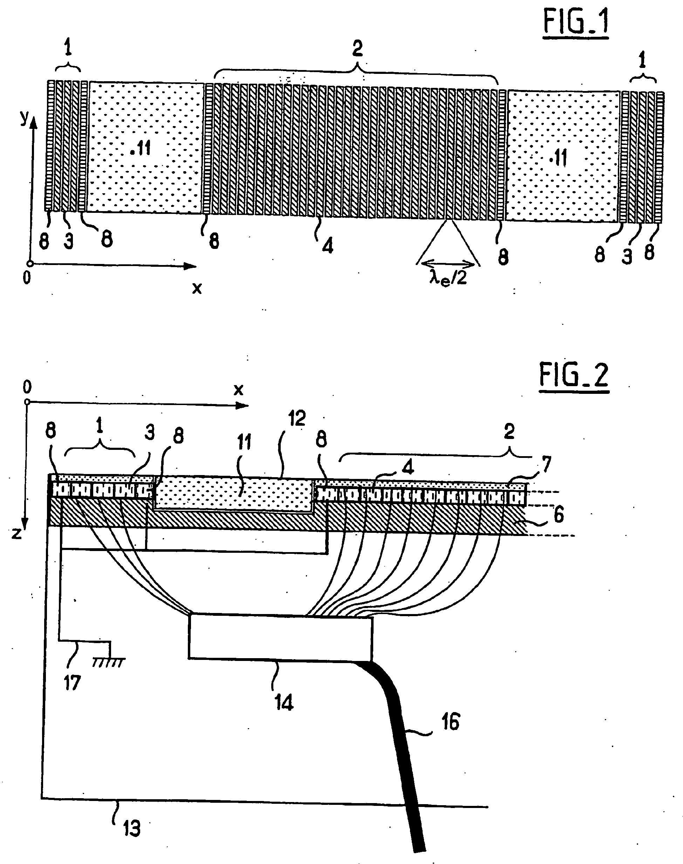

Comme le montrent les figures 1 et 2, la sonde est de type barrette linéaire constituée de transducteurs ultrasonores piézoélectriques plans, non focalisés. Les transducteurs sont répartis en trois zones, à savoir une zone de réception centrale 2 située entre deux zones d'émission 1. La zone de réception 2 comprend des transducteurs 4 équidistants fonctionnant en récepteurs et chaque zone d'émission 1 comprend des transducteurs 3 équidistants fonctionnant en émetteurs. Intrinsèquement, tout transducteur peut être soit émetteur, soit récepteur, et sa fonction effective dans la sonde ne dépend que du dispositif émetteur/récepteur électronique sur lequel on branche la sonde, et qui sera décrit en détail plus loin en tant que partie de l'appareil d'évaluation selon l'invention.

La sonde est dite unidimensionnelle, en ce sens que tous les transducteurs 3, 4 sont alignés selon une seule et même rangée rectiligne, et sont disposés dans un plan commun qui correspond au plan de la figure 1. D'une manière qui est classique pour les barrettes échographiques disponibles dans le commerce, les transducteurs 3, 4, sont disposés entre un milieu arrière 6 (figure 2) dit « backing », et une lame adaptatrice ou « face avant » 7 destinée au contact avec le corps (la peau) du patient, avec interposition d'un gel.

Dans chaque application, les dimensions de la surface de chaque élément transducteur sont déterminées par la directivité et la sensibilité des éléments. Les transducteurs ont de préférence une largeur égale à $\lambda_e/2$, expression dans laquelle $\lambda_e$ est la longueur d'onde dans l'eau des ultrasons à la fréquence centrale de la sonde. Cette largeur permet d'obtenir une sonde à large ouverture angulaire. La largeur des transducteurs est orientée parallèlement à la direction d'alignement Ox des transducteurs 3 et 4 le long de la sonde. Dans chaque zone, les transducteurs 3 ou 4 sont adjacents les uns aux autres. La longueur de chaque transducteur 3 et 4, mesurée dans le plan des transducteurs (plan de la figure 1) mais suivant la direction Oy perpendiculaire à leur direction d'alignement Ox, est choisie suffisamment grande pour obtenir un élément suffisamment sensible. Dans l'exemple non-limitatif de la figure 1, la longueur des transducteurs est égale à quatre fois $\lambda_e$.

La fréquence centrale utilisée peut être comprise par exemple entre 100 kHz et 5 MHz, non limitativement, pour une sonde utilisée sur le corps humain ou animal. Si la fréquence centrale est de 1 MHz, on a $\lambda_e = 1{,}5$ mm. On appelle fréquence centrale de la sonde la fréquence calculée d'après le passage du signal par deux zéros consécutifs dans le même sens, c'est à dire par exemple en allant d'une valeur positive à une valeur négative de l'intensité.

Chaque zone 1 ou 2 est encadrée par deux éléments 8 reliés à la masse. Ainsi, lorsqu'ils sont activés, les transducteurs 3 et 4 qui sont en limite de région 1 ou 2 fonctionnent dans les mêmes conditions qu'un transducteur 3 ou 4 situé entre deux autres transducteurs de la même région. Il est en outre prévu entre la région de réception 2 et chacune des deux régions d'émission 1 une barrière absorbante 11 qui dans l'exemple représenté est réalisée en liège. Les barrières 11 dites « barrières de face avant », sont destinées à atténuer fortement les ondes susceptibles de se propager dans la face avant de la sonde. Elles forment chacune une interruption dans la lame adaptatrice 7 (figure 2), s'étendent dans toute la zone d'épaisseur (suivant la direction Oz) des transducteurs 3 et 4, et font saillie dans le milieu arrière 6 sans toutefois le traverser complètement. Chaque barrière 11 présente une face antérieure 12 destinée à n'être séparée du contact avec la peau du patient que par un film d'étanchéité qui recouvre également la lame 7. La face 12 est plane et coplanaire avec la face frontale de la lame adaptatrice 7. Chaque barrière 11 présente en direction Oy une dimension au moins égale aux transducteurs, c'est à dire dans l'exemple quatre fois $\lambda_e$. Dans la direction Ox, la barrière 11 s'étend sur par exemple 10 mm.

La sonde étant dans l'air, pour un signal électrique de 160 Volts émis sur un transducteur d'un côté d'une barrière 11, le signal électrique reçu par un transducteur situé de l'autre côté de la barrière est inférieur à 160 microvolts, de sorte que l'atténuation en amplitude est au moins égale à un facteur $10^6$, ce qui équivaut à 120 dB.

Dans l'exemple représenté à la figure 1, la zone de réception comprend 32 transducteurs 4 adjacents les uns aux autres, et chaque zone d'émission comprend trois transducteurs 3 adjacents les uns aux autres. Il y a donc en tout 38 transducteurs, six éléments 8 reliés à la masse et deux barrières 11, et ainsi la longueur totale de la sonde est de l'ordre de 50 mm avec les dimensionnements exposés plus hauts. En pratique, la zone de réception peut typiquement, mais non limitativement, comporter 10 à 64 récepteurs selon l'utilisation de la sonde et la taille de sonde souhaitée. Le choix dépend d'un compromis entre la fréquence centrale choisie et le site du squelette qui est exploré.

Il est théoriquement possible de prévoir un seul émetteur 3 dans chaque zone d'émission 1. Il est toutefois préféré d'en prévoir plus qu'un et par exemple trois comme dans l'exemple représenté, pour deux raisons différentes. La première raison est que si un émetteur 3 est défectueux, la sonde est encore utilisable avec les deux autres émetteurs. Un autre avantage de prévoir plusieurs émetteurs est de permettre de faire fonctionner simultanément au moins deux émetteurs 3 de la même zone d'émission 1 en les calant temporellement de telle manière qu'ils soient en concordance spatiale de phase. Autrement dit, lors d'une émission, le signal émis par l'émetteur 3 le plus proche de la zone de réception vient se superposer en concordance de phase avec le signal venant de plus loin, le long de l'interface à ausculter, en provenance de l'autre émetteur en fonctionnement, plus éloigné de la zone de réception 2.

La sonde comprend encore un boîtier plastique de protection 13 qui n'est représenté que schématiquement à la figure 2. Le film d'étanchéité recouvrant la lame adaptatrice 7 et la face antérieure 12 des barrières absorbantes Il est apparent sur toute une face antérieure du boîtier. Du côté opposé à la sonde proprement dite, le boîtier 13 renferme un connecteur 14 par lequel les transducteurs 3 et 4 sont raccordés à un câble unique 16 dans lequel les liaisons électriques individuelles avec chaque transducteur sont rassemblées spatialement. Dans la représentation schématique de la figure 2, la masse 17 est représentée à l'intérieur du boîtier 13, mais en pratique, il s'agit d'un fil supplémentaire dans le câble 16, par l'intermédiaire du connecteur 14.

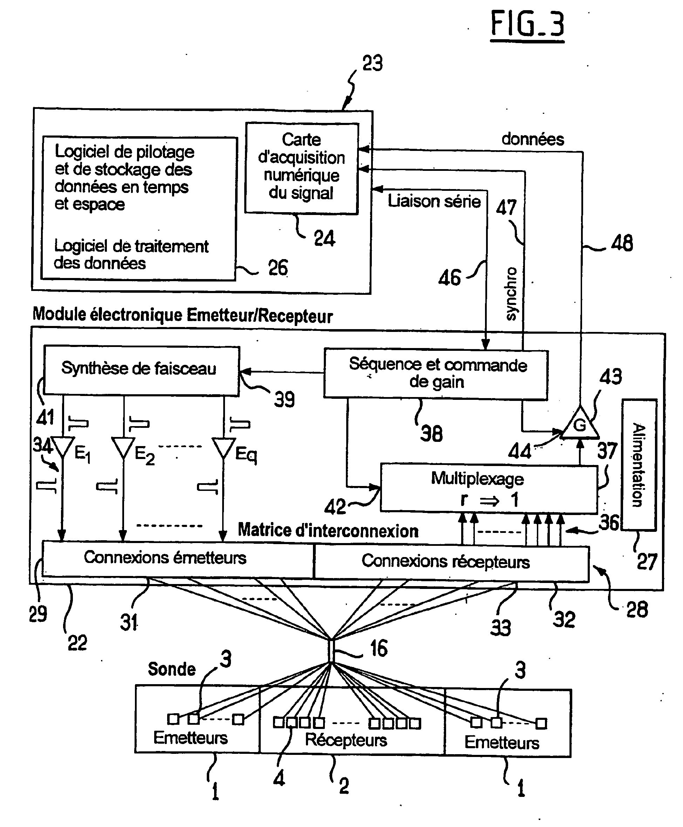

Comme le montre la figure 3, l'appareil selon l'invention pour évaluer in vivo et de manière non-invasive les propriétés mécaniques d'un os, comprend :

- une sonde 21 qui peut être telle que décrit en référence aux figures 1 et 2 et qui n'est que très schématiquement représentée ;
- un module électronique émetteur/récepteur 22 qui est relié d'une part à la sonde 21 par le câble 16 et d'autre part à un ordinateur PC 23 dans lequel sont installées une carte d'acquisition numérique du signal 24 ainsi qu'une partie logicielle 26 permettant de commander la carte d'acquisition numérique du signal 24 et le module électronique émetteur/récepteur 22. Le logiciel 26 assure également le stockage des données en temps et en espace, et le traitement de ces données pour fournir les évaluations attendues. Les données en temps et en espace précitées comprennent en particulier la localisation des émetteurs et des récepteurs activés ainsi que les instants d'émission et de réception correspondants.

Le module électronique 22 contient une partie émission (partie gauche à la figure 3) et une partie réception (partie droite à la figure 3), ainsi qu'une alimentation 27. Le module électronique comprend en particulier une matrice d'interconnexion 28 comprenant elle-même une partie émission 29 munie de bornes telles que 31 auxquelles sont reliées, respectivement, les liaisons du câble 16 raccordées aux émetteurs 3 de la sonde 21, et une partie réception 32 comportant des bornes 33 auxquelles sont reliées les liaisons du câble 16 qui sont raccordées aux récepteurs 4 de la sonde 21.

La partie émission du module comporte ainsi q voies indépendantes émettrices 34, par exemple seize voies dans une réalisation typique, par lesquelles des excitations électriques d'amplitude 160 V sont envoyées aux émetteurs 3 de la sonde 21. Les seize voies peuvent émettre soit une à la fois, soit plusieurs à la fois avec des retards relatifs réglables de façon à réaliser une « synthèse de faisceau », c'est à dire la concordance spatiale de phase décrite précédemment. La partie réception comporte r voies 36, par exemple 64 dans une réalisation typique, qui raccordent chaque récepteur 4 de la sonde 21, via la partie réception 32 de la matrice d'interconnexion 28, avec un multiplexeur 37 qui permet la lecture successive des r voies 36 par la carte d'acquisition 24.

Il y a encore dans le module électronique émetteur/récepteur un étage séquenceur et de commande de gain 38 qui est relié à une entrée de commande 39 d'un étage de synthèse de faisceau 41 d'où partent les voies émettrices 34. Par l'entrée de commande 39, le séquenceur 38 commande l'étage 41 de façon à définir d'une part les instants des tirs (émissions de signal par les émetteurs 3 de la sonde 21), et d'autre part les émetteurs concernés par le tir à effectuer à chaque instant de tir.

Le séquenceur 38 est également relié à une entrée de commande 42 du multiplexeur 37 pour définir, à chaque instant, le numéro de la voie réceptrice 36 dont l'état d'excitation doit être transmis à un amplificateur de sortie 43.

L'étage séquenceur et de commande de gain 38 est en outre raccordé à une entrée de commande 44 pour commander le gain G de l'amplificateur de sortie 43.

Les liaisons entre l'ordinateur PC 23 et le module électronique 22 comprennent :

- une liaison 46, typiquement mais non-limitativement une liaison série, avec le séquenceur 38, permettant au logiciel 26 de programmer les séquences de tirs à effectuer par les émetteurs 3, ainsi que la position du récepteur 4 dont le signal doit être transmis à l'entrée de l'amplificateur 43 pour chaque tir, respectivement ;
- une liaison de synchronisation 47 par laquelle la carte d'acquisition 24 est synchronisée avec les tirs réglés par le séquenceur 38 ; et
- une liaison données 48 entre la sortie de l'amplificateur 43 du module 22 et la carte d'acquisition 24, pour permettre la lecture et l'enregistrement des données dans l'ordinateur PC 23.

Le procédé que l'on va décrire plus loin pour déterminer la vitesse de propagation du signal ultrasonore le long de la surface d'un os nécessite de déterminer l'écart entre les temps de parcours respectifs du signal jusqu'à chacun d'au moins deux récepteurs 4 de la sonde. Comme le module électronique 22 ne surveille qu'un seul récepteur 4 à la fois, on détecte l'écart de temps de parcours précité non pas par détection de l'arrivée du signal consécutif à un même tir successivement sur les deux détecteurs, mais par une méthode d'échantillonnage spatio-temporel consistant à effectuer deux tirs différents en activant pour chaque tir l'un respectif des deux récepteurs, après quoi on peut calculer l'écart de temps de parcours du signal entre les deux récepteurs en calculant la différence entre les temps de parcours respectifs depuis l'émetteur jusqu'à chacun des récepteurs.

En outre, pour connaître le diagramme espace-temps d'une particularité de signal le long de l'os, c'est à dire le diagramme donnant la distance parcourue par cette particularité le long de l'interface en fonction du temps, on effectue des tirs respectifs pour chacun des récepteurs 4 de façon à obtenir, dans l'exemple d'une sonde à 32 récepteurs, 32 points du diagramme espace-temps.

Par ailleurs, pour minimiser les effets des aléas de mesure, chaque mesure du temps de parcours entre un ou plusieurs émetteurs et un récepteur 4 déterminé est effectuée n fois et on prend comme temps de parcours le temps de parcours mesuré sur la moyenne des n tirs.

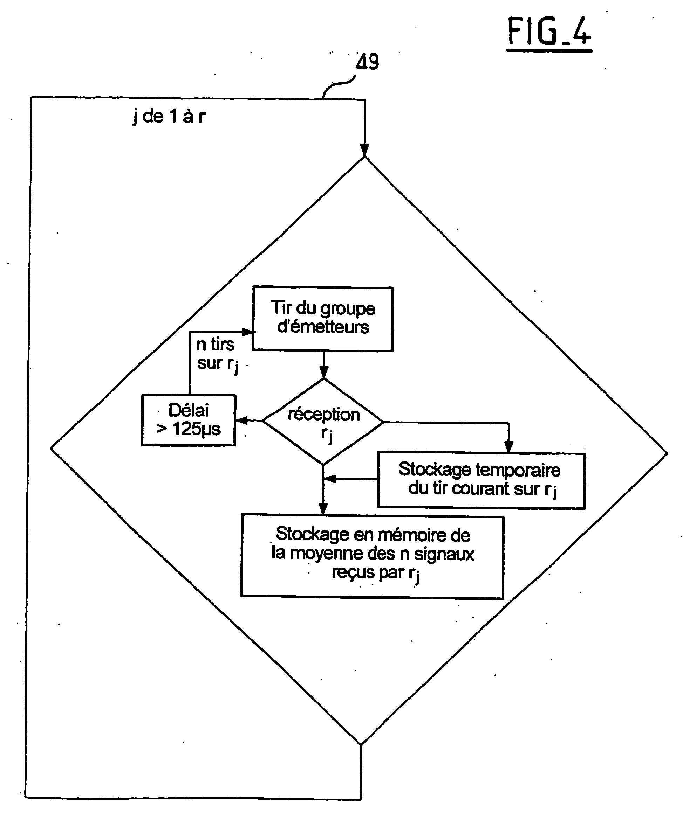

Ce processus est visualisé à la figure 4. Une boucle principale 49 est parcourue pour chacune des r voies de réception 36. Pour chaque récepteur 4 ayant la position j (j variant de 1 à r), on réalise deux séries de n tirs. La première série de tirs correspond à l'émission par un groupe d'émetteurs 3 situés d'un côté de la zone de réception, la seconde série correspondant à l'émission par l'autre groupe d'émetteurs 3 situé de l'autre côté de la zone de réception 1. Pour chaque récepteur 4, et pour une zone d'émission, le système émet n tirs dont on fait la moyenne, et on enregistre alors le tir moyen résultant de la moyenne des n tirs. Cette moyenne permet une réduction du bruit aléatoire. Au total, un cycle de mesure correspond à 2.r.n tirs qui conduisent à l'enregistrement de 2r signaux temporels moyennés. On peut en outre réaliser autant de cycles successifs que désiré, en précisant un nombre p de cycles de mesure dans le logiciel 26 de pilotage de l'appareil de la figure 3. On obtient alors un fichier final de données contenant 2.r.p signaux temporels moyennés. A la fin de chaque série de r.n tirs les données sont transférées de la carte 24 vers la mémoire vive de l'ordinateur 23. L'ensemble des données est laissé dans la mémoire vive tant que l'ensemble des p cycles n'est pas terminé. A la fin des p cycles, les données sont enregistrées sur un fichier. A la fin de chaque série de r.n tirs (2.p séries au total), on dispose donc des données en mémoire vive pour un éventuel traitement en temps réel.

Le logiciel de pilotage 26 installé dans l'ordinateur 23 pour piloter le module électronique 22 permet de spécifier tous les paramètres nécessaires à la définition d'une acquisition complète :

- le nom du fichier d'enregistrement et le chemin d'accès dans l'ordinateur ;
- le choix des émetteurs 3 et des récepteurs 4 mis en jeu ;
- les éventuels retards relatifs entre les tirs d'un groupe d'émetteurs 4 afin de réaliser l'effet de synthèse de faisceau décrit précédemment : on règle les retards relatifs d'après l'effet constaté sur les récepteurs (4), où on recherche une superposition des signaux correspondant aux émetteurs simultanément activés ;

- le gain associé à chaque récepteur (4), car il est par exemple nécessaire de prévoir un gain plus fort pour les récepteurs (4) les plus éloignés du groupe d'émetteurs (3) qui est activé, ou parce qu'un gain plus fort est nécessaire pour certains récepteurs (4) moins sensibles, ce qui peut être déterminé par des tests préalables, ou encore en fonction des individus ;
- le nombre de tirs (n) à moyenner pour chaque récepteur;
- le nombre de cycles (p) d'une acquisition complète ;
- le délai éventuel entre ces cycles ;
- le temps entre les tirs successifs ;
- la fréquence d'échantillonnage, c'est-à-dire la cadence à laquelle sont relevés les états d'excitation successifs du récepteur 4 qui est activé. Cette fréquence est au moins égale à 10 fois, de préférence 50 fois la fréquence centrale du signal ultrasonore émis ;
- la durée des signaux acquis (donc le nombre de points d'acquisition par signal temporel), ce qui est un paramètre significatif lorsqu'on souhaite relever des informations en plus de celles du délai d'arrivée du premier signal.

Le dispositif a été optimisé de façon à pouvoir réaliser le plus rapidement possible un grand nombre de mesures. Le temps minimum entre deux tirs successifs est par exemple de 125$\mu$s dans une réalisation typique. Ce temps minimum peut être dicté par un délai de rechargement d'un condensateur, ou par un délai de disparition du signal ultrasonore précédent. A titre d'exemple, le temps mis pour réaliser un cycle de mesure complet avec n = 10 tirs par récepteur, et si la sonde comporte 50 récepteurs 4, est typiquement de 125$\mu$s x 2 x 10 x 50 = 125 ms. Par conséquent, même si la sonde est tenue manuellement par un opérateur sur le corps d'un patient, celle-ci est quasiment immobile pendant un cycle de mesure.

Pour chaque tir, l'état d'excitation du récepteur 4 qui est activé est relevé et enregistré à chaque instant d'échantillonnage. On obtient donc une base de valeurs donnant l'état d'excitation du récepteur en fonction du temps pendant la durée de la mesure relative à ce tir, par exemple 125 $\mu$s. Comme instant d'arrivée du premier signal, on peut prendre le premier instant d'échantillonnage où l'état d'excitation dépasse un certain niveau prédéterminé, le premier passage par un maximum, le premier changement de signe, etc. Une fois les tirs effectués, on ramène tous les relevés à la même origine des temps et on fait la moyenne des n tirs en calculant le niveau moyen d'excitation de chaque récepteur à chaque instant d'échantillonnage. On obtient ainsi un relevé moyen sur lequel on recherche, par exemple, la particularité de signal servant de critère pour l'arrivée du premier signal.

Le procédé de relevage qui vient d'être décrit permet aussi de réaliser une « image » spatio-temporelle des modes d'excitation qui se propagent le long de l'interface en conséquence d'un tir. L'image spatio-temporelle au sens de la présente invention est une matrice bi-dimensionnelle des états d'excitation en fonction du récepteur, chaque position de récepteur correspondant par exemple à une colonne de la matrice, et chaque instant d'échantillonnage (identifié par son retard par rapport à l'instant de tir) correspondant par exemple à une ligne de la matrice.

Pour chaque récepteur, donc pour chaque colonne dans l'exemple, la succession de valeurs utilisées peut être, suivant les applications :

- Les valeurs relevées au cours de l'un quelconque par exemple le premier des n tirs dans un sens donné pour ce récepteur ;
- Les valeurs relevées pour ce récepteur dans un sens au cours de celui des n tirs qui correspond le mieux à la valeur moyenne calculée pour le temps d'arrivée du premier signal ;
- Pour chaque instant la moyenne des valeurs d'excitation relevées pour cet instant au cours des n tirs dans un sens de propagation.

On peut encore, non seulement pour l'arrivée du premier signal mais aussi pour chaque point du signal, calculer par le procédé selon l'invention le temps mis par ce point du signal pour se propager d'un récepteur à l'autre et obtenir une image spatio-temporelle débarrassée de la déformation introduite par le milieu intercalaire.

L' « image » ainsi obtenue par l'une ou l'autre méthode fait apparaître le mode « transmission directe » du signal le long de l'os, et des modes induits dits « longitudinaux », correspondant à des vibrations en compression-détente avec des efforts parallèles à la direction de propagation, et des modes induits dits transverses, correspondant à des contraintes de cisaillement entre des plans perpendiculaires à la direction de propagation. L'image spatio-temporelle est décalée et éventuellement déformée par l'effet des tissus mous, mais sa configuration reste identifiable et analysable à des fins de diagnostic.

Le type d'acquisition (temps/espace) permet donc de réaliser une analyse (fréquence temporelle)-(fréquence spatiale) en ce sens que les lignes (dans l'exemple précité) de la matrice donnent une image des états d'excitation de la rangée de récepteurs à un instant donné, et les colonnes (toujours dans l'exemple précité) de la matrice donnent une image des états d'excitation successifs d'un récepteur donné dans le temps qui suit un tir.

L'image spatio-temporelle est obtenue avec des moyens très simples puisqu'un seul récepteur est activé à chaque instant. On appelle échantillonnage spatio-temporel le procédé utilisé, qui consiste à ne relever l'état d'excitation que d'un petit nombre de détecteurs (un seul détecteur dans l'exemple préféré) pour chaque tir (échantillonnage spatial) et à relever pour ce détecteur l'état d'excitation à des instants successifs à la suite de ce tir (échantillonnage temporel), puis à obtenir « l'image spatio-temporelle », c'est-à-dire la matrice bidimensionnelle en ramenant tous les relevés à la même origine des temps, par exemple l'instant de tir.

Lors de la réalisation de p cycles de mesure successifs, le système offre la possibilité d'obtenir une valeur en temps réel de la vitesse du premier signal, calculée à partir de deux séries successives de r.n tirs, à savoir une série dans chaque sens, les données associées à un cycle étant présentes dans la mémoire vive de l'ordinateur 23. Le temps entre deux tirs successifs est également réglable entre le minimum technique précité de par exemple 125μs, à une valeur plus élevée, par exemple pour l'enregistrement de signaux au-delà de 125μs ou la visualisation en temps réel de la vitesse en fonction du cycle. Ceci peut permettre entre autres de visualiser en temps réel la vitesse du premier signal mesurée en fonction de la position de la sonde sur l'os, pour repérer par exemple une zone de vitesse extrémale.

Le logiciel de traitement des données utilise les données lues par la carte d'acquisition 24, soit en post-traitement à partir des fichiers de données enregistrées sur le disque dur de l'ordinateur, soit directement après chaque cycle à partir de la mémoire vive de l'ordinateur pour des traitements en temps réel. L'utilisation de tirs ultrasonores dans deux sens opposés vers les mêmes récepteurs 4 permet de calculer la vitesse d'un signal le long de l'interface de réfraction sous-jacente, c'est-à-dire dans l'application considérée à la surface de l'os, indépendamment de l'effet des tissus mous qui sont interposés entre l'interface et la sonde, et indépendamment de l'effet d'une éventuelle inclinaison entre la direction étudiée de l'interface et le plan des récepteurs.

On va exposer ci-après ce procédé d'élimination de l'effet des tissus mous et de l'inclinaison en se référent à la propagation du premier signal mais le procédé est applicable aux autres types de signaux qui sont produits dans l'os pour un tir composé par un train d'alternances individuelles.

On va d'abord décrire comment à partir d'une paire de récepteurs 4 ayant entre eux une distance connue et qui reçoivent l'onde rayonnée par l'interface dans un sens de propagation puis dans l'autre, on peut obtenir par un calcul très simple la vitesse de l'onde dans l'os, c'est-à-dire principalement dans la couche superficielle de l'os adjacente à l'interface avec les tissus mous, indépendamment de l'épaisseur des tissus mous et même d'une éventuelle inclinaison α entre le plan des deux récepteurs composant la paire, et la direction de propagation étudiée le long de la surface de l'os.

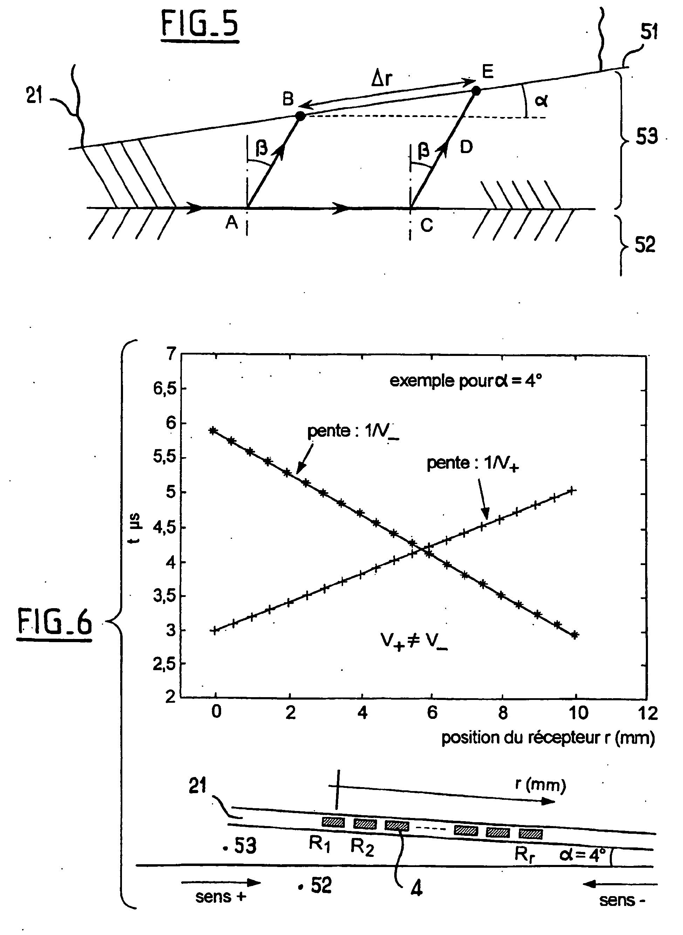

On a représenté à la figure 5 une partie de la sonde 21 placée sur la surface de la peau 51 d'un patient dont l'os à ausculter est désigné par 52 et les tissus mous interposés entre la sonde et l'os sont désignés par 53. Des traits plus épais montrent les trajets géométriques suivis par l'onde arrivant en deux récepteurs situés en B et E, séparés d'une distance Δr. L'onde se propageant le long de l'os 52 est en même temps renvoyée par réfraction à travers les tissus mous vers la sonde suivant un angle β qui, de manière connue, est donné par la relation

$$Sin\beta = \frac{V_{T_m}}{V_{os}} \hspace{12em} (1)$$

dans laquelle $V_{Tm}$ et $V_{os}$ sont respectivement la vitesse du signal dans les tissus mous 53 et dans l'os 52.

L'angle critique β ainsi déterminé détermine aussi les points A et C d'où partent, le long de l'os 52, les signaux réfractés qui seront reçus par les récepteurs situés en B et E respectivement.

Par ailleurs, la différence de temps d'arrivée aux deux récepteurs s'écrit :

$$\Delta t = (t_{AC} + t_{CD} + t_{DE}) - t_{AB} = t_{AC} + t_{DE}$$

On sait également que $t_{AC} = \frac{BD}{V_{os}}$ et que $t_{DE} = \frac{DE}{V_{Tm}}$

Le point D est le point de la droite du segment CE tel que CD = AB.

Les différentes relations qui viennent d'être exposées permettent de déduire après quelques manipulations trigonométriques l'expression suivante :

$$\Delta t = \frac{\Delta r.\cos(\alpha)}{V_{Os}} \cdot (1 + \frac{\tan(\alpha)}{\tan(\beta)}) .$$

Si l'onde arrive dans l'autre sens le long de la surface de l'os, il suffit de changer le signe de $\alpha$ dans l'expression précédente. Finalement, l'écart de temps d'arrivée s'écrit, pour les deux sens de propagation:

$$\Delta t^{\pm} = \frac{\Delta r.\cos(\alpha)}{V_{Os}} \cdot (1 \pm \frac{\tan(\alpha)}{\tan(\beta)}) \qquad (2)$$

l'indice $\pm$ correspondant aux deux sens possibles de propagation le long de la surface.

L'intérêt de ce procédé de tir dans les deux sens vers les deux mêmes récepteurs est de fournir très simplement la vitesse dans l'os à partir de $\Delta t^+$ et $\Delta t^-$ : en sommant $\Delta t^+$ et $\Delta t^-$, on obtient simplement :

$$V_{os} \simeq \frac{2.\Delta r}{\Delta t^+ + \Delta t^-} \times \cos(\alpha) \qquad (3)$$

L'angle $\alpha$ est inconnu a priori lors d'une expérience *in vivo*, mais on peut néanmoins raisonnablement le supposer petit, et écrire $\cos(\alpha) \cong 1$, à l'ordre 2 en $\alpha$. On a alors très simplement comme première approximation $V_{osa0}$ de la vitesse de l'onde dans l'os la relation :

$$V_{Os a0} = \frac{2.\Delta r}{\Delta t^+ + \Delta t^-} \cong V_{Os} \qquad (4)$$

par exemple, pour $\alpha < 4°$, l'erreur relative commise est inférieure à $1-\cos(\alpha)=0.2\%$. Si toutefois une meilleure exactitude est requise, on peut utiliser la valeur $V_{osao}$ pour estimer une valeur $\alpha_1$ de $\alpha$. En effet, d'après l'expression (2), on déduit :

$$\tan(\alpha_1) = 2\left[\frac{\Delta t^+ - \Delta t^-}{\Delta t^+ + \Delta t^-}\right] \tan \beta_1 \qquad (5)$$

expression dans laquelle $\tan(\beta_1)$ est une estimation de $\tan(\beta)$ à partir de l'expression (1). D'après la relation (3) on peut alors recalculer une valeur plus précise

$$V_{osa1} = V_{osa0} \times \cos(\alpha_1)$$

de la vitesse de propagation recherchée. Dans la relation (1), on ne connaît pas la valeur exacte de la vitesse $V_{Tm}$ dans les tissus mous, mais on sait qu'elle est toujours voisine d'une valeur approchée $Vt_{me}$ de 1500 m/s. En pratique, il suffit de la prendre toujours égale à 1500 m/s pour obtenir une estimation suffisamment précise de $\alpha$. La correction de la vitesse approchée par l'estimation de $\alpha$ est en fait assez peu sensible aux valeurs exactes de $Vt_m$ et $V_{os}$ utilisées dans le calcul de $\beta$, comme l'illustre l'exemple numérique suivant:

Exemple numérique:

Supposons que $Vt_m = 1600$ m/s, que $\alpha = 4°$, $\Delta r = 0.8$ mm, et que la vitesse des ondes dans l'os soit $V_{os} = 4000$ m/s. Les formules théoriques exactes (2) donnent $\Delta t^+ = 0.231479..\mu s$ et $\Delta t^- = 0.167546...\mu s$, qui conduisent à $V_{osao} = 4009.8$ m/s, soit une erreur relative de 0.24%. L'estimation de a par (5), avec $V_{tme} = 1500$ m/s, au lieu de $Vt_m$ 1600 m/s et $V_{osa0}$ au lieu de $V_{os}$ donne $\alpha = 3.70°$, proche de la valeur réelle. En reportant dans (3), on obtient $V_{osa1} = 4001.4$ m/s, soit une erreur inférieure à 0.04%. On obtient donc une très bonne précision, sans connaître l'angle $\alpha$ et en utilisant une valeur estimée très approximative grossière de $Vt_{me} = 1500$ m/s au lieu de la valeur réelle $Vt_m = 1600$ m/s.

Si l'on souhaite encore améliorer la précision, on peut obtenir une valeur plus précise $\beta_2$ de l'angle $\beta$ en utilisant la valeur $V_{osa1}$ dans l'expression (1), puis obtenir une valeur plus précise $\alpha_2$ de l'angle a en utilisant la valeur $\beta_2$ dans l'expression (5), et calculer une valeur plus précise $V_{osa2} = V_{osa0} \times \cos(\alpha_2)$,
et ainsi de suite itérativement :

$$V_{osak} = V_{osa0} \cos (\alpha_k)$$

Un intérêt du procédé selon l'invention est de fournir une estimation très précise de la vitesse le long de la surface de l'os, en ne connaissant que très approximativement la vitesse dans les tissus mous, vitesse qu'il suffit de prendre égale à 1500 m/s (vitesse approximative dans les tissus mous donnée dans la littérature). La vitesse dans l'os est obtenue très simplement en calculant la somme $\Delta t^+ + \Delta t$ des différences de temps d'arrivée dans les deux sens, avec une approximation à l'ordre 2 en $\alpha$ nettement suffisante pour des mesures *in vivo.* (Si une précision supérieure est nécessaire, on peut l'obtenir par estimation de $\alpha$ comme décrit précédemment). Cette correction de l'angle $\alpha$ n'est possible que parce que la zone de réception est commune aux tirs dans les deux sens, condition nécessaire pour que l'angle $\alpha$ entre les récepteurs utilisés et la surface de l'os soit le même pour les deux tirs dans les sens opposés.
La sonde préférentielle décrite possède un groupe de récepteurs 4, plutôt qu'une paire unique de récepteurs, afin d'obtenir une meilleure précision sur la mesure. La façon la plus simple de calculer la vitesse corrigée pour éliminer l'effet de l'angle a entre l'alignement des récepteurs et la surface de la sonde, est de tracer le graphe des temps d'arrivée aux récepteurs en fonction de la position des récepteurs, ceci pour les tirs dans les deux sens, comme le montre figure 6, où les récepteurs 4 sont désignés individuellement par R1 et Rr. On obtient deux séries de points, qui s'alignent sur deux droites de pentes

$$\frac{1}{V^+} = \frac{\Delta t^+}{\Delta r} \quad \text{et} \quad \frac{1}{V^-} = \frac{\Delta t^-}{\Delta r} .$$

qui ont des valeurs absolues différentes si $\alpha$ est non nul, ce qui est le cas illustré à la figure 6. La vitesse corrigée (à l'ordre 2 en $\alpha$) est simplement obtenue en procédant à des substitutions dans l'expression (4) qui devient :

$$V_{Os\,ao} = \frac{2}{\dfrac{\Delta t^+}{\Delta r} + \dfrac{\Delta t^-}{\Delta r}} \cong V_{Os} \qquad\qquad (6)$$

valeur qui peut être précisée par le procédé itératif déjà décrit. Ce procédé de mesure par la pente fournit une mesure plus robuste qu'une simple mesure de $\dfrac{\Delta t^+}{\Delta r}$ et $\dfrac{\Delta t^-}{\Delta r}$ obtenue à partir de deux récepteurs, puisqu'on intègre un plus grand nombre de récepteurs, ce qui compense les incertitudes de mesure si celles-ci sont de nature aléatoire. Ce procédé donne des résultats très précis si les capteurs sont parfaitement alignés sur une même ligne, et que leurs positions relatives sur cette ligne sont connues avec précision (même si ils ne sont pas également répartis). La précision relative nécessaire sur les positions des récepteurs est d'au moins 1%, si l'on souhaite obtenir la vitesse $V_{os}$ avec moins de 1% d'erreur. Or il est technologiquement difficile de positionner les récepteurs avec une telle précision à la fabrication. Pour y remédier, il est préféré, selon l'invention, de calibrer la sonde, de préférence suivant un procédé de calibrage de la sonde, utilisant la correction d'angle précédemment décrite.

Procédé de calibrage de la sonde.

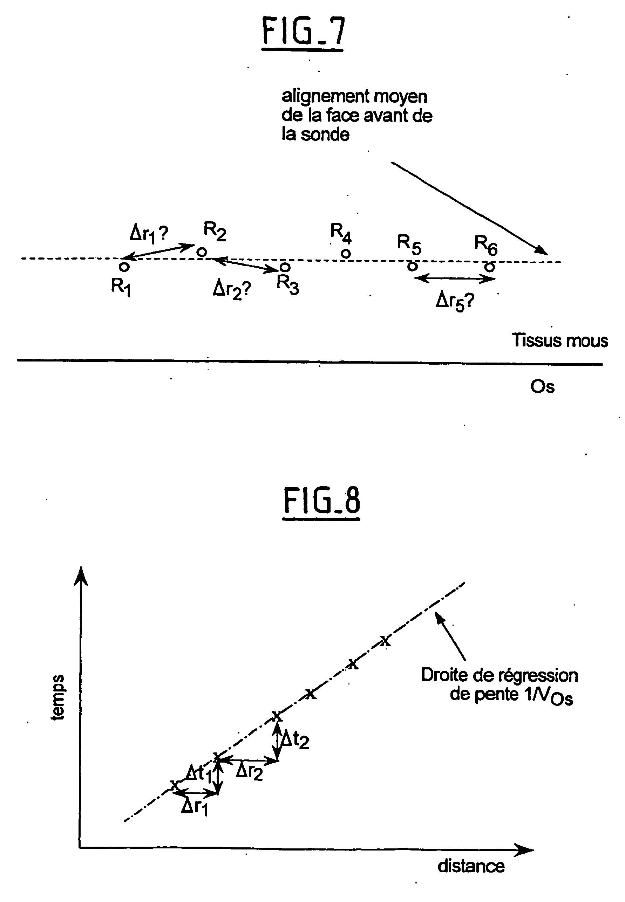

Le problème de la position relative des récepteurs 4 dans la sonde 21 est de deux sortes: les distances entre récepteurs adjacents sont inconnues d'une part, et d'autre part l'angle que fait une paire de récepteurs adjacents avec la surface de l'os dépend de la paire considérée. Ceci est illustré à la figure 7, avec six récepteurs. Le procédé de base décrit jusqu'à présent corrige automatiquement l'angulation de chaque paire, mais ne corrige pas les incertitudes sur la distance entre récepteurs successifs. Le principe du calibrage que l'on va maintenant décrire consiste en la détermination de la distance entre les deux récepteurs de chaque paire. En pratique, on place la sonde en regard d'un matériau calibré dans lequel la vitesse de propagation est connue précisément, avec l'alignement moyen des récepteurs à peu près parallèle à la surface du matériau (de façon que tous les angles considérés soient petits). On mesure alors les différences $\Delta t^+$ et $\Delta t^-$ de temps d'arrivée pour chaque paire de récepteurs adjacents. Pour chaque paire de récepteurs adjacents, la différence de temps d'arrivée qui serait obtenue si l'angle de la paire avec la surface était nul vaut

$$\Delta t = \frac{\Delta t^+ + \Delta t^-}{2}$$ (à l'ordre 2 en $\alpha$). Connaissant la vitesse $V_{Ca1}$, qui vaut $V_{cal} = \frac{\Delta r}{\Delta t}$, on en déduit pour chaque

paire la distance $\Delta r = V_{cal} \times \Delta t$ qui sépare les 2 récepteurs.

Lors d'une mesure sur une surface osseuse pour laquelle on désire déterminer la vitesse de propagation, on commence par déterminer les durées de parcours corrigées $\Delta t = \frac{\Delta t^+ + \Delta t^-}{2}$. Les distances $\Delta r$ étant par ailleurs

connues par calibrage, on peut alors disposer les points de mesure sur un graphique (temps-distance), de façon que chaque paire de récepteurs adjacents soit séparés de At selon l'axe des temps et $\Delta r$ selon l'axe des distances. La vitesse est alors obtenue par simple mesure de la pente de la droite de régression passant par l'ensemble des points (voir figure 8). Dans cette version du procédé, c'est à dire avec calibrage préalable, l'angle $\alpha$ qui n'a pas besoin d'être connu, est différent pour chaque paire de détecteurs considérés en raison de l'incertitude sur le positionnement exact de chaque détecteur.

En résumé, le procédé selon l'invention utilisant le principe de tirs dans deux sens opposés vers les mêmes récepteurs le long de la surface permet d'obtenir une vitesse corrigée de l'effet des tissus mous, par un calcul très simple. De plus contrairement aux dispositifs nécessitant un parallélisme entre la surface de la sonde et la surface de l'os, ce procédé permet d'obtenir une vitesse automatiquement corrigée, quelle que soit la position de la sonde par rapport à la surface osseuse. Ce calcul de vitesse de premier signal peut-être effectué et affiché en temps réel, entre p cycles de mesures successifs. L'utilisateur peut choisir le délai entre deux cycles successifs, de façon à rechercher manuellement en temps réel une zone de l'os où la vitesse est maximum, par exemple. La signification physique de la vitesse de premier signal corrigée de l'effet des tissus mous est discutée ci-après en ce qui concerne les applications.

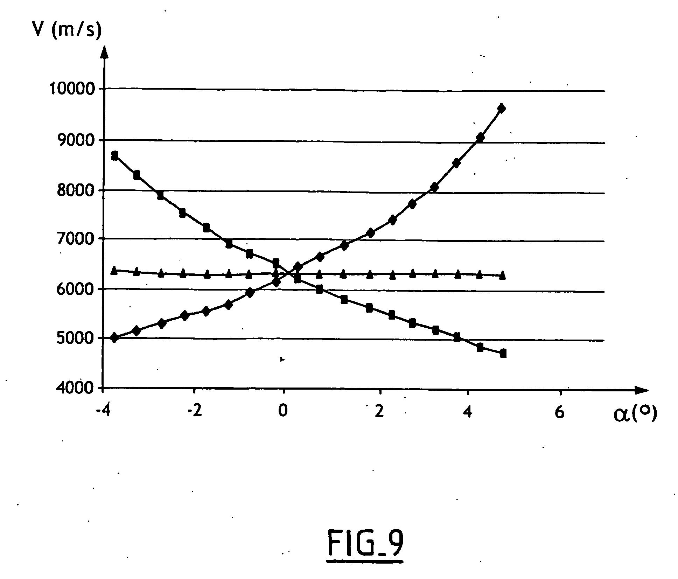

La figure 9 illustre, sur un échantillon d'aluminium, la correction de l'effet de l'angle. Pour un angle $\alpha$ donné entre la sonde et l'échantillon, allant de - 4° à + 4°, les valeurs $V^+$ et $V^-$ obtenues par régression linéaire (figure 8) s'échelonnent entre 5000 et 9000 m/s. Mais la vitesse corrigée obtenue avec l'expression (6) précédemment décrite a une stabilité relative meilleure que 0,2%.

## Applications

### Application à l'étude de l'os

Selon l'OMS (Organisation Mondiale de la Santé), l'ostéoporose est une maladie systémique du squelette (affectant l'ensemble du squelette) caractérisée par une masse osseuse basse et une détérioration de la micro-architecture de l'os, entraînant une augmentation de la fragilité osseuse et du risque de fracture. Sur l'os cortical, ceci se traduit par une augmentation de la porosité de l'os, ainsi que par une diminution de l'épaisseur corticale. La propagation des ondes ultrasonores dans la couche corticale, sensible aux propriétés mécaniques locales de l'os et à sa géométrie, est donc modifiée par l'atteinte ostéoporotique.

Il a en particulier été montré que la vitesse du premier signal se propageant le long de la surface corticale du radius distal est un paramètre qui permet de discriminer une population saine d'une population ayant subi une fracture de la hanche [M. Weiss, A. Ben-Shlomo, P. Hagag and S. Ish-Shalom, "Discrimination of proximal hip fracture by quantitative ultrasound measurement at the radius", Osteoporosis Int. 11, 411-416 (2000)]. La signification physique de la vitesse de premier signal dépend de la longueur d'onde dans l'os à la fréquence centrale considérée et de l'épaisseur corticale. Pour des épaisseurs corticales supérieures typiquement à la longueur d'onde, la vitesse de premier signal corrigée de l'effet des tissus mous est égale à la vitesse des ondes longitudinales de volume dans l'os [E. Bossy, M. Talmant, P.Laugier, "Axial transmission of 1 MHz ultrasonic waves on thin cortical bone plates: a simulation study.", IEEE UFFC 2001 International Conférence]. Cette vitesse est donc indépendante de l'épaisseur de l'os cortical et ne dépend que de la densité et de l'élasticité du tissu osseux.

Pour des épaisseurs corticales petites devant la longueur d'onde, la vitesse de premier signal corrigée de l'effet des tissus mous est égale à la vitesse de propagation du mode de Lamb symétrique $S_0$.

L'utilisation de l'invention sur l'os n'est pas restreinte à la détection des modifications ostéoporotiques, mais peut être étendue à l'étude de toute maladie osseuse affectant les propriétés mécaniques de l'os et sa géométrie. Elle peut également être étendue à la pédiatrie, pour le suivi du développement osseux chez l'enfant.

**Application à d'autres matériaux**

L'utilisation de la présente invention n'est pas limitée à l'étude de l'os. On peut également étudier tous les matériaux présentant une surface sur laquelle on peut apposer la sonde et le gel de couplage, et qui possèdent au moins une vitesse de propagation plus grande que celle du milieu intercalaire. On peut ainsi appliquer la méthode de mesure proposée à des matériaux de type bois, métaux, polymères plastiques, verre. Par exemple, on peut par mesure de vitesse du premier signal mesurer la vitesse des ondes longitudinales dans des pièces de roseaux utilisée dans la fabrication d'anches pour instruments de musique, afin de discriminer des anches défectueuses.

**Vérifications Expérimentales**

*Montage Expérimental*

Chaque sonde consiste en une barrette unidimensionnelle linéaire de transducteurs à large bande (largeur relative de la bande de fréquence : 70%) avec deux groupes d'émetteurs encadrant un groupe unique de 14 récepteurs. Une sonde fonctionne à une fréquence centrale de 1 MHz, tandis que l'autre sonde fonctionne à une fréquence centrale de 2 MHz. Tous les éléments identiques ont une largeur $\lambda/2$, et sont séparés par un pas inter-élémentaire légèrement supérieur à $\lambda/2$, $\lambda$ étant la longueur d'onde dans le tissu mou, proche de la longueur d'onde dans l'eau. Ceci assure à la fois pour les émetteurs et les récepteurs un large diagramme angulaire qui inclut les angles critiques associés aux ondes de volume en compression dans l'os, ces angles critiques étant prévus compris dans la gamme allant de 21-26°. Les dimensions hors-tout des sondes ne dépassent pas 3-5 cm le long de l'axe longitudinal de la sonde (axe X de la figure 1) et 1 cm dans sa direction transversale (axe Y de la figure 1). Pour les deux sondes, la distance couverte par les récepteurs le long de l'axe de l'os est d'environ 1 cm, et la distance minimale entre les émetteurs et les récepteurs est d'environ 15 mm. Sur le radius, une telle distance permet prévisiblement l'observation de l'onde rayonnée provenant de l'os en tant que premier signal arrivant avant toute autre contribution originaire de la propagation à travers le tissu mou. Des distances minimales plus faibles peuvent être envisagées pour des sites squelettiques avec une moindre épaisseur de tissu mou entre la sonde et l'os, tels que les phalanges ou le métacarpe. Les sondes sont fabriquées par Vermon, 180 rue du Général RENAULT, 37000 Tours, France.

Les émetteurs étaient excités avec une fréquence maximale de répétition d'impulsion de 10 kHz avec une demi-période électrique de 160 V en impulsion carrée. Le premier signal détectable atteint les récepteurs typiquement entre 5 et 10 $\mu$s après l'émission, avec une amplitude mesurée de l'ordre de 1 mV. La mesure d'un signal de si faible amplitude (en comparaison avec la tension entrante) juste après l'émission nécessite un fort découplage entre l'émission et la réception. L'électronique dédiée a été réalisé par Ultrasons Technology, Boulevard TONNELLE, 37000 Tours, France.

Les signaux étaient numérisés sur un convertisseur analogique numérique à 12 bits (fabriqué par Signatec Inc., Sheridan street, Suite 117, Corona, 91720 Californie, Etats-Unis), avec une fréquence d'échantillonnage typique de 50 MHz pour la sonde à 1 MHz, et 100 MHz pour la sonde à 2 MHz.

Le traitement de signal nécessite la détection du premier signal arrivant (PSA) sur les récepteurs, pour en déduire sa vitesse. Ceci a été réalisé en calculant la durée entre les signaux atteignant deux récepteurs adjacents en mesurant et soustrayant leurs temps de parcours respectifs. Le PSA était défini comme étant la première contribution se distinguant du bruit, le temps de parcours étant défini d'après le positionnement temporaire du premier extremum du signal. Une détermination précise de ce temps de parcours a été obtenue au moyen d'une interpolation parabolique du signal au voisinage de l'extremum.

La vitesse de l'onde rayonnée a été trouvée en utilisant une combinaison des modes de transmission directe et inverse (c'est à dire dans les deux sens opposés) suivant l'invention. Pour chaque paire de récepteurs, deux durées ont été déduites des mesures de temps de parcours : la première appelée $\Delta t^+$ a été obtenue par le mode de transmission directe, et la seconde, appelée At-, a été obtenue par le mode de transmission inverse. On a ensuite obtenu la valeur moyennée corrigée $\Delta t_{corr} = \dfrac{\Delta t^- + \Delta t^+}{2}$. En utilisant deux récepteurs, la vitesse de phase de l'onde rayonnée est obtenue par la relation suivante :

$$C_{Ph} \cong \frac{\Delta r}{\Delta t_{corr}} \qquad\qquad (7)$$

Cette équation (7) est équivalente à l'équation (4) ci-dessus. La barrette linéaire de récepteurs, comprenant 14 récepteurs, forme 13 paires de récepteurs adjacents et la relation (7) ci-dessus a été appliquée à chacune de ces paires. On a formé par régression linéaire la courbe représentant les durées successives $\Delta t_{Corr}$ par rapport à la distance entre les récepteurs de chaque couple respectif pour obtenir l'inverse de la vitesse du PSA corrigée à l'égard de l'inclinaison entre la sonde et la surface mesurée.

### *Echantillons mesurés*

Des séries d'expériences ont été effectuées sur des plaques épaisses en Polyméthacrylate (matériau connu sous la marque Plexiglas ou Perspex) et en Aluminium. L'épaisseur de la plaque était suffisamment grande pour que la mesure de l'onde frontale de compression revienne à mesurer le premier signal détecté sur les récepteurs. Toutefois, le principe de mesure peut être appliqué à toute onde détectable rayonnée de la surface de l'os. Ces matériaux d'essai ont été choisis car ils ont une vitesse d'onde de volume en compression qui est respectivement inférieure et supérieure à la vitesse d'environ 3500-4200 m/s usuellement trouvée dans l'os cortical humain, (voir R. Barkmann, E. Kantorovich, C. Singal, D. Hans, H.K. Genant, M. Heller, C.C. Glüer : « A new method for quantitative ultrasond measurements at multiple skeletal sites, » Journal of Clinical Densitometry, vol. 3, pp. 1-7, 2000 ; et M. R. Stegman, R.P. Heaney, D. Travers-Gustafson, J. Leist, « Cortical ultrasound velocity as an indicator of bone status » Osteoporosis International, vol. 5, pp. 349-353, 1995). Pour des spécimen plongés dans l'eau, les angles critiques sont environ de 13° et 33° pour l'Aluminium et le Plexiglas respectivement, alors que les valeurs correspondantes pour l'os cortical sont d'environ 21-26°. Les expériences avec des plaques académiques ont également été effectuées avec des échantillons plongés dans du glycérol à 98%, dans lequel les ultrasons se propagent à environ à 1900 m/s. On a choisi le glycérol pour son atténuation ultra-sonore d'environ 0,2 dB/cm à 1 MHz (voir Handbook of Physical Quantities, Chapitre 7), proche des valeurs d'atténuation présentées par la peau humaine.

### *Calibrage des sondes.*

La précision des évaluations de vitesse dépend d'un bon calibrage des sondes. Un tel calibrage nécessite que la vitesse de l'onde mesurée soit connue sur un matériau de référence. On a utilisé un échantillon de Plexiglas dont la vitesse de l'onde de volume en compression est de $2770 \pm 10$ m/s à 18 °C (mesurée indépendamment au laboratoire par un procédé de substitution classique) pour calibrer les sondes des présents essais. Ce calibrage consistait à déterminer avec précision la distance $\Delta r$ entre récepteurs consécutifs. $\Delta r$ a été obtenue pour chaque paire de récepteurs consécutifs en mesurant $\Delta t_{Corr}$ et en utilisant l'équation (7), dans laquelle $C_{ph}$ est la vitesse de référence connue du matériau de calibrage.

### *Essais*

Pour les essais où les échantillons sont plongés dans de l'eau où du glycérol, les sondes étaient montées sur un système de déplacement mécanique, permettant la translation verticale (axe Z de la figure 1) par pas de 10 µm. Il était possible de faire pivoter la sonde autour de l'axe transversal Y par pas angulaires de 5'. Ces rotations correspondent à l'angle $\alpha$ appelé précédemment angle d'inclinaison en référence à la figure 5. On peut également effectuer une rotation autour de l'axe longitudinal X, autrement dit une rotation de roulis. Pour les essais en contact, on a utilisé un gel ultrasonore standard comme agent de couplage. Les expériences impliquant des mesures des effets de la température ont été effectuées dans le même récipient, après chauffage du bain. On a considéré que l'équilibre thermique local était atteint sur le site de la mesure à chaque instant. On contrôlait la température au moyen d'un thermocouple.

### *Erreurs de mesure*

On a défini l'erreur de mesure comme étant la moitié de l'amplitude de dispersion des mesures obtenues sur un échantillon pour une procédure de mesure donnée. Les erreurs de précision sont données sous forme de valeurs absolues :

$$Erreur = \frac{C_{max} - C_{min}}{2}$$

ou sous forme de valeur relative :

$$Erreur\ relative\ [\%] = \frac{C_{max} - C_{min}}{2.c_{réel}} \cdot 100$$

Comme la valeur réelle de la vitesse ultrasonore était inconnue dans les essais effectués, on a pris $C_{réel}$ comme étant la valeur moyenne mesurée obtenue à partir de plusieurs mesures.

***Résultats***

Correction à l'égard de l'inclinaison

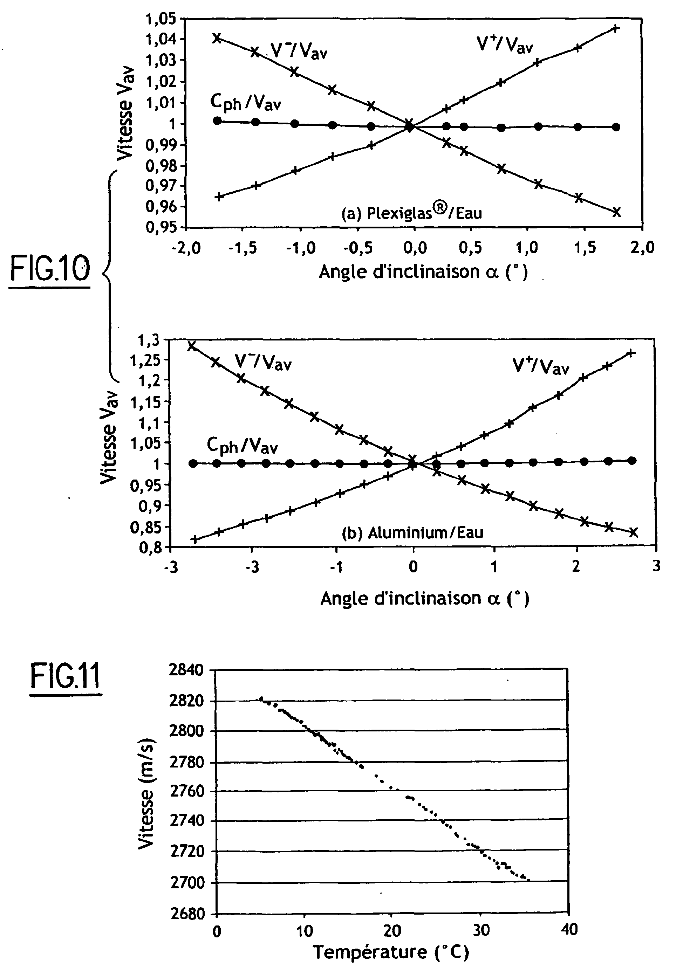

Pour ces premiers essais, on a plongé les échantillons de Plexiglas et d'aluminium dans l'eau. La sonde était placée environ 3 mm au-dessus de l'interface, avec un angle d'inclinaison $\alpha$ variant typiquement de -2° à + 2° par rapport à la surface de l'échantillon. En utilisant le procédé de correction décrit plus haut, on a déterminé la vitesse du PSA en fonction de l'angle d'inclinaison. En outre on a déterminé les vitesses apparentes $V^+$ et $V^-$ dans les modes de transmission directe et inverse. La figure 10 représente les vitesses apparentes et corrigées $c_{ph}$ (données par l'équation (7)) normalisées par la vitesse corrigée moyenne $V_{av}$ pour le Plexiglas et l'aluminium obtenues lors de mesures à 1MHz. Les deux graphiques montrent que lorsque l'angle d'inclinaison augmente, l'écart entre les valeurs apparentes $V^+$ et V- par rapport à la valeur moyenne augmente. En outre, conformément à l'équation (2), plus la vitesse réelle est grande (c'est à dire plus l'angle critique est faible), plus l'erreur est elle-même importante : pour un angle d'inclinaison de 2°, l'erreur est de l'ordre de 150 m/s (erreur relative de 5%) et de 900 m/s (erreur relative de 15%) pour le Plexiglas et l'aluminium respectivement.

Le Tableau 1 ci-après montre l'erreur subsistante après la correction à l'égard de l'inclinaison selon l'invention pour les échantillons de Plexiglas et d'aluminium plongés dans l'eau, avec la sonde à 1 MHz et la sonde à 2 MHz.

Tableau 1 : Précision de la vitesse du PSA corrigée à l'égard de l'inclinaison de la sonde

| | Eau/Plexiglas | | Eau/aluminium | |
|---|---|---|---|---|
| Fréquence centrale | 1 MHz | 2 MHz, | 1 MHz | 2 MHz |
| Angle d'inclinaison (°) | -1,7 à +1,8 | -2,5 à +2,5 | -2,7 à +2,7 | -2,4 à +2,1 |
| Vitesse moyenne du PSA (m/s) | 2769 | 2768 | 6036 | 6198 |
| Gamme de vitesse du PSA (m/s) | 2767-2774 | 2764-2772 | 6027-6043 | 6191-6215 |
| Precision relative (%) | 0,13 | 0,15 | 0,14 | 0,19 |

L'erreur subsistante après la correction à l'égard de l'inclinaison apparaît inférieure à 5 m/s, ce qui correspond à une erreur relative de 0,15% pour le Plexiglas, et inférieure à 17 m/s, correspondant à une erreur relative de 0,25% pour l'aluminium. Les essais ainsi effectués valident la correction proposée à l'égard de l'inclinaison.

On a aussi investigué les effets d'autres causes potentielles d'erreur qui peuvent être rencontrées *in vivo*.

Autres sources potentielles d'erreur

Les Tableaux 2 et 3 ci-dessous montrent l'influence de la distance entre la sonde et l'interface sur les évaluations de vitesse. Le Tableau 2 concerne le cas où l'échantillon est plongé dans l'eau alors que le Tableau 3 concerne le cas où l'échantillon est plongé dans le glycérol.

Tableau 2 : Influence de la distance entre la sonde et l'interface (cas eau/solide) sur les évaluations de vitesse

| | Eau / Plexiglas | | Eau / aluminium | |
|---|---|---|---|---|
| Fréquence centrale | 1 MHz | 2 MHz | 1 MHz | 2 MHz |
| Distance sonde échantillon (mm) | 0,5 - 4,0 | 0,5 - 3,5 | 0,5-4,5 | 0,5 - 4,0 |
| Vitesse moyenne du PSA (m/s) | 2761 | 2762 | 6034 | 6206 |

(suite)

| | Eau / Plexiglas | | Eau / aluminium | |
|---|---|---|---|---|
| Fréquence centrale | 1 MHz | 2 MHz | 1 MHz | 2 MHz |
| Gamme de vitesses de PSA (m/s) | 2754-2764 | 2757-2767 | 6030-6037 | 6195-6216 |
| Précision rélative (%) | 0,18 | 0,19 | 0,06 | 0,17 |

Tableau 3 : Influence de la distance entre la sonde et l'interface (cas glycérol/solide) sur les évaluations de vitesse

| | Glycérol / Plexiglas | | Glycérol/Aluminium |
|---|---|---|---|
| Fréquence centrale | 1 MHz | 2 MHz | 1 MHz |
| Distance sonde échantillon (mm) | 0,5-3,5 | 0,5-4,0 | 0,5-5,0 |
| Vitesse moyenne du PSA (m/s) | 2744 | 2753 | 5994 |
| Gamme de vitesses de PSA (m/s) | 2732-2747 | 2742-2757 | 5976-6011 |
| Précision relative (%) | 0,28 | 0,26 | 0,29 |

Pour les expériences *in vivo,* l'épaisseur de tissu mou sous la sonde dépend du patient ainsi que du site du squelette où est effectuée la mesure. Selon les équations (4) et (7), la vitesse corrigée $V_{osa0}$ ou $C_{ph}$, ou même la vitesse plus précise de l'équation (3) qui est égale à $V_{os} = V_{osa0}$ x cos (a), est indépendante des propriétés du tissu mou, et en particulier de son épaisseur. Pour vérifier expérimentalement cette affirmation, des mesures de vitesse ont été effectuées sur plusieurs matériaux d'essai en faisant varier la distance sonde/échantillon avec deux fluides de couplage différents : l'eau et le glycérol. Pour éviter les éventuels effets de confusion entre différents facteurs, on a maintenu la sonde parallèle à l'interface mesurée. Comme le montre le Tableau 2, une fois toutes les données collectées, les erreurs relatives maximales ressortent inférieures à 0,3% pour les deux expériences, dans l'eau et dans le glycérol. On a fait varier la distance entre la sonde et l'échantillon entre 0,5 et 5 mm.

La comparaison des résultats des Tableaux 2 (concernant le cas où le fluide de couplage est l'eau) et 3 (cas où le fluide de couplage est le glycérol) montre que les vitesses mesurées sont légèrement inférieures lorsque le fluide de couplage est le glycérol, que lorsque ce fluide est l'eau.

On a également étudié l'effet du roulis de la sonde autour de l'axe longitudinal X. On a étudié l'influence du roulis sur la vitesse mesurée pour simuler les situations que l'on peut rencontrer *in vivo* sur des sites osseux corticaux courbes, tels que le radius ou les phalanges, où la sonde ne peut pas toujours être précisément positionnée perpendiculairement à la surface osseuse sur le site de mesure. Pour des angles de roulis inférieurs à 2°, l'erreur relative sur la vitesse du PSA s'est avérée inférieure à 0,05%, quatre fois moins que les effets de l'inclinaison. On peut donc considérer que cet effet est négligeable.

Enfin on a testé la précision (c'est à dire la reproductibilité) des mesures de contact lorsque la sonde est couplée avec l'échantillon par un gel ultrasonore standard. On a effectué quatre mesures successives séparées par des repositionnements et l'erreur relative associée à cette procédure de mesure est apparue égale à 0,05% pour les expériences sur le Plexiglas et 0,08% sur l'aluminium. Comme la sonde n'a plus aucun degré de liberté, ces valeurs pour des mesures en contact correspondent à la meilleure reproductibilité possible de la technique sur un échantillon plat.

Sensibilité

La haute précision des mesures de vitesse fait que les sondes selon l'invention sont des outils puissants pour étudier des changements subtils qui peuvent survenir dans un matériau. L'un des buts ultimes est d'utiliser cette mesure pour détecter les changements subtils dans l'os, causés par l'ostéoporose ou en réponse à un traitement. Par exemple, on illustre ici les performances de la sonde en étudiant la sensibilité de la technique aux effets de température dans le Plexiglas. On a effectué une série de mesures sur une plaque de Plexiglas plongée dans l'eau, dont on fait varier la température. La Figure 11 illustre les variations de la vitesse de l'onde frontale lorsque la température varie de 5 à 40°C. Dans la gamme de températures étudiée, les mesures obtenues montrent une décroissance linéaire de 4,25 m.s$^{-1}$/°C

EP 1 507 475 B1

de la vitesse lorsque la température augmente, conformément à une publication antérieure (D.L. Folds, « Expérimental détermination of ultrasonic wave velocities in plastics, elastomers, and synthetic foam as a function of temperature, » J. Acoust. Soc. Am., vol. 52, pp. 426-427, 1972). Cette expérience démontre la grande sensibilité du dispositif pour mesurer les petites variations de vitesse.

## DISCUSSION

L'invention vise un nouveau procédé, une nouvelle sonde et un nouvel appareillage appropriés pour l'étude *in vivo* des propriétés de l'os cortical. La conception et le dimensionnement de la sonde, en particulier la distance minimale source-récepteur, tient compte de l'épaisseur de tissu mou recouvrant l'os cortical, de sorte que le premier signal détecté corresponde à une onde rayonnée de l'os. La technique permet la correction automatique à l'égard des effets du tissu mou qui recouvre l'os dans la région de la mesure. On n'a besoin de mesurer ni la vitesse dans le tissu mou ni l'épaisseur du tissu mou. En premier lieu, en supposant que l'épaisseur du tissu mou est constante sous la sonde, le signal peut être transmis dans une direction et la vitesse le long de l'os peut être obtenue d'après la différence d'un temps de parcours du premier signal arrivant sur une paire de détecteurs, en particulier de détecteurs adjacents, normalisée à la distance entre ces détecteurs. Dans une telle procédure de mesure, deux trajets acoustiques sont impliqués, comportant des trajets partiels de même longueur dans le tissu mou, et un trajet partiel de longueur variable le long de l'os. Le second niveau de correction est obtenu pour tenir compte de l'épaisseur variable du tissu mou. En supposant une variation linéaire de l'épaisseur du tissu mou entre les deux détecteurs, la correction à l'égard du tissu mou se base désormais sur une moyenne du temps de parcours de signaux qui ont été émis de part et d'autre de la paire de récepteurs adjacents et se sont propagés le long de l'os cortical dans des directions opposées. A cette fin, la barrette de transducteurs est composée d'un groupe de récepteurs encadré par deux émetteurs ou deux groupes d'émetteurs. La correction est locale et appropriée pour la partie de l'os qui est en-dessous de la paire de transducteurs récepteurs. Elle peut être étendue à chaque paire de transducteurs adjacents dans la sonde et peut donc être appliquée pour corriger les effets de l'épaisseur variable dans des cas d'interface légèrement courbe se traduisant par une épaisseur de tissu mou qui varie de manière non-uniforme le long de la surface de l'os, à la seule supposition que l'épaisseur de tissu mou varie linéairement en fonction de la distance sous chaque paire de récepteurs. Etant donné la faible distance entre récepteurs adjacents et la forme tout de même quasi-plane de l'interface, cette supposition apparaît raisonnable et permet de traiter la plupart des situations rencontrées *in vivo* à la surface de la diaphyse des os longs. En outre, on peut obtenir un profil de vitesse unidimensionnel sous la zone de récepteurs, ce qui permet par exemple le suivi de la guérison d'une fracture ou la caractérisation d'un défaut local quelconque.

Les mesures de vitesse sur matériau d'essai sont ainsi validées expérimentalement. Les essais ont été effectués sur des échantillons épais de sorte que le premier signal arrivant (PSA) correspond toujours à l'onde frontale de compression. Mais le mode de mesure selon l'invention peut être appliqué à toute onde détectable, ou toute particularité d'onde détectable provenant de la surface de l'os. Parmi les diverses sources d'erreurs de mesure qui peuvent affecter la mesure de la transmission axiale, l'effet de l'inclinaison de la sonde est apparu comme étant le plus important. Le mode de correction proposé élimine toute erreur due au tissu mou et conduit à des mesures précises de la vitesse du son avec une erreur maximum de 0,3% en précision relative, alors que des erreurs d'au moins 3% ont été trouvées sur le Plexiglas dès que la sonde a une inclinaison d'au moins 1° par rapport à la surface de l'os. L'erreur est encore plus grande lorsque l'échantillon est en aluminium conformément à l'équation (2) : plus le milieu de propagation est rapide plus les mesures sont faussées lorsque l'invention n'est pas appliquée. Dans les essais effectués, les effets de toutes les autres causes potentielles d'erreurs étudiées sur les mesures de vitesse se sont avérées être inférieures d'au moins un ordre de grandeur par rapport à la source principale d'erreur, celle due à l'inclinaison.

Néanmoins, les valeurs de vitesse obtenues à 1 MHz (6034 m/s) et à 2 MHz (6206 m/s) sont significativement différentes, alors que les deux sondes ont été calibrées sur un échantillon de Plexiglas. Le calibrage a été effectué en supposant que la vitesse de l'onde frontale était égale à la vitesse de l'onde de volume dans le Plexiglas. Mais cette supposition peut ne pas être toujours valable et on doit être prudent en interprétant les mesures de vitesse.

Des résultats obtenus par des simulations numériques bi-dimensionnelles (voir E. Bossy, M. Talmant, et P. Laugier, « Effect of bone cortical thickness on velocity measurements using ultrasonic axial transmission : a 2D simulation study », J. Acoust. -Soc. Am., vol. 112, pp. 297-307, 2002) montrent que cette supposition est valable avec une précision relative supérieure à 0,1% uniquement lorsque le rapport de la distance à la longueur d'onde est typiquement supérieur à 10. Pour des distances de propagation plus courtes, de l'ordre de quelques longueurs d'onde, la vitesse de l'onde frontale est inférieure de quelques pourcents à la vitesse de l'onde de compression. En ce qui concerne la longueur d'onde de compression dans l'aluminium, la sonde de 1 MHz utilisée pour les essais est plus courte que la sonde à 2 MHz, ce qui peut expliquer la différence observée. Cette discussion montre qu'il y a des difficultés potentielles à comparer les valeurs absolues obtenues à partir de dispositifs différents (fabricant différent, fréquences différentes) pour mesurer les propriétés de l'os au moyen de la transmission axiale. En particulier, un calibrage préliminaire commun sur du Plexiglas ne garantirait pas que des dispositifs différents donneraient des résultats absolus similaires sur l'os cortical car les distances entre émetteurs et récepteurs, mesurées en longueurs d'onde, différeraient d'un dispositif à l'autre. De préférence, le calibrage doit être effectué sur un matériau de référence ayant une vitesse mesurée proche de celle de l'os cortical.

Les résultats du Tableau 2 (l'eau constituant le milieu de couplage) et 3 (le glycérol constituant le milieu de couplage) suggèrent que la grande viscosité du glycérol réduit légèrement la vitesse mesurée pour le premier signal arrivant, comparée aux valeurs de vitesse mesurées lorsqu'on utilise l'eau. Néanmoins cette diminution relative reste de l'ordre de la précision permise pour la correction d'inclinaison.

**Revendications**

1. Procédé pour évaluer de manière non invasive une durée de parcours et/ou une vitesse de propagation de signaux ultrasonores entre deux points le long d'une interface dans un corps, en particulier le long d'une surface osseuse, dans lequel, au moyen d'émetteurs (3) et de récepteurs (4) d'ultrasons placés à la surface accessible du corps le long de l'interface à ausculter, et répartis le long d'une lame adaptatrice, on détermine pour chacun des deux sens de propagation opposés le long de l'interface l'écart de temps de parcours jusqu'à au moins deux récepteurs espacés le long de la direction de propagation étudiée, **caractérisé en ce qu'**on utilise des émetteurs (3) placés de part et d'autre des au moins deux récepteurs, de façon que les mêmes récepteurs reçoivent les signaux pour les deux sens de propagation.

2. Procédé selon la revendication 1, **caractérisé en ce que** lesdits au moins deux récepteurs (4) forment une rangée suivant la direction (Ox) de propagation et sont allongés transversalement à la direction de propagation étudiée, en étant sensiblement équidistants le long de ladite direction.

3. Procédé selon la revendication 1 ou 2, **caractérisé en ce que** la vitesse de propagation du signal le long de l'interface est calculée de manière approchée d'après la formule

$$V_{osa0} \cong 2 \frac{\Delta r}{\Delta t^+ + \Delta t^-}$$

dans laquelle $V_{osaO}$ est la valeur approchée de la vitesse de propagation du signal, $\Delta r$ est la distance entre les deux récepteurs, $\Delta t^+$ et $\Delta t^-$ sont, pour chaque sens de propagation, respectivement, l'écart de temps de parcours du signal jusqu'au deux récepteurs.

4. Procédé selon la revendication 1 ou 2, **caractérisé en ce que** lorsqu'on utilise une rangée de récepteurs (41) en nombre supérieur à 2, on procède aux calculs suivants :

   - on détermine pour chaque sens de propagation la pente d'une droite représentative des temps de parcours d'un signal en fonction de la position des récepteurs le long de la rangée ;
   - on détermine la vitesse approchée du signal dans l'os d'après la formule :

$$V_{osa0} \cong \frac{2}{\frac{1}{V^+} + \frac{1}{V^-}}$$

dans laquelle

$\frac{1}{V^+} = \texttt{pente}$ de la courbe sensiblement rectiligne du temps de parcours du signal en fonction de la position du récepteur dans la rangée dans un sens de propagation ;

$\frac{1}{V^-} = \texttt{pente}$ de la courbe sensiblement rectiligne du temps de parcours du signal en fonction de la position du récepteur dans la rangée dans l'autre sens de propagation.

**5.** Procédé selon la revendication 3 ou 4, **caractérisé en ce qu'**on accroît la précision de la détermination de la vitesse de propagation du signal par au moins une itération comprenant les trois calculs suivants, dans lesquels k représente le rang de l'itération:

a) évaluation $\beta_k$ de l'angle $\beta$ selon lequel le signal traverse le milieu intercalaire situé entre l'interface et les récepteurs par la formule :

$$Sin\beta_k \cong \frac{V_{tme}}{V_{osa(k-1)}}$$

dans laquelle :

$V_{tme}$ = vitesse supposée du signal dans le milieu intercalaire ;
$V_{osa(k-1)}$ = dernière vitesse estimée de la propagation le long de l'interface.

b) évaluation $\alpha_k$ de l'angle d'inclinaison ($\alpha$) de l'interface par rapport à la rangée de récepteurs en fonction de l'angle $\beta_k$ obtenu à l'étape (a) :

$$\tan(\alpha_k) = 2\left[\frac{\Delta t^+ - \Delta t^-}{\Delta t^+ + \Delta t^-}\right]\tan\beta_k$$

c) détermination de la nouvelle valeur approchée de la vitesse de propagation.

$$V_{osak} = V_{osa0}\ Cos\alpha_k$$

**6.** Procédé selon l'une des revendications 1 à 5, **caractérisé en ce que** pour chaque sens de propagation on détermine ledit écart de temps de parcours en faisant la différence ($\Delta t^+$, $\Delta t^-$) entre le temps de parcours d'au moins un premier signal jusqu'à un premier des au moins deux récepteurs, et le temps de parcours d'au moins un deuxième signal jusqu'à un deuxième des au moins deux récepteurs.

**7.** Procédé selon la revendication 6, **caractérisé en ce que** les signaux sont séparés par des interruptions.

**8.** Procédé selon la revendication 6 ou 7, **caractérisé en ce que** pour chaque signal on active un seul récepteur (4).

**9.** Procédé selon l'une des revendications 1 à 8, **caractérisé en ce qu'**on utilise des récepteurs (4) ayant entre eux un écartement qui est compris entre environ 100 $\mu$m et environ 1 cm.

**10.** Procédé selon l'une des revendications 1 à 9, **caractérisé en ce qu'**on utilise un signal dont la demi-longueur d'onde dans l'eau ($\lambda_e/2$) correspond approximativement à la dimension de chaque récepteur (41) dans la direction de propagation étudiée (Ox).

**11.** Procédé selon l'une des revendications 1 à 10, **caractérisé en ce que** pour un même sens de propagation on utilise au moins deux émetteurs (3) que l'on synchronise pour émettre en concordance spatiale de phase.

**12.** Procédé selon l'une des revendications 1 à 11, **caractérisé en ce qu'**on utilise des récepteurs reliés (4) entre eux de manière sensiblement fixe dans une sonde (21).

**13.** Procédé selon la revendication 12, **caractérisé en ce qu'**on calibre la sonde (21) en procédant à une mesure préalable de l'écart de temps de parcours de signaux jusqu'au détecteurs successifs, dans les deux sens, le long d'une interface de référence placée parallèlement à la direction moyenne d'alignement des récepteurs, puis en calculant la distance $\Delta r$ entre deux récepteurs en fonction de la vitesse de propagation connue $V_{cal}$ le long de l'interface et des écarts de temps $\Delta t^+$ et $\Delta t^-$ dans les deux sens de propagation par la formule $\Delta r = Vcal (\Delta t^+ + \Delta t^-)/2$.

**14.** Procédé selon l'une des revendications 1 à 13, **caractérisé en ce qu'**il est appliqué à la détermination de la vitesse du premier signal reçu en réponse à un signal émis.

**15.** Procédé selon l'une des revendications 1 à 13, **caractérisé en ce qu'**il est appliqué pour débarrasser de la déformation temporelle due au milieu intercalaire, une image spatio-temporelle des signaux reçus par au moins deux détecteurs en réponse à un signal émis.

**16.** Sonde à ultra-sons pour l'auscultation osseuse pour la mise en oeuvre d'un procédé selon l'une des revendications 1 à 15, comprenant des émetteurs (3) et des récepteurs (4) d'ultrasons, répartis le long d'une lame adaptatrice, **caractérisée en ce que** les émetteurs (3) sont placés dans deux zones d'émission (1) situées de part et d'autre d'une zone de réception (2) où se trouvent les récepteurs (4) de façon que les mêmes récepteurs reçoivent les signaux pour les deux sens de propagation.

**17.** Sonde selon la revendication 16, **caractérisée par** une barrière (11) absorbante pour les ultra-sons entre la zone de réception (2) et chacune des zones d'émission (1).

**18.** Sonde selon la revendication 17, **caractérisée en ce que** la barrière (11) est en liège.

**19.** Sonde selon la revendication 17 ou 18, **caractérisée en ce que** la barrière (11) s'étend sensiblement jusqu'à une face (12) pour le contact avec le corps du patient.

**20.** Sonde selon l'une des revendications 18 à 20, **caractérisée en ce que** la barrière (11) s'étend vers l'arrière, en faisant saillie dans un milieu arrière (6) formant support commun aux émetteurs (3) et aux récepteurs (4).

**21.** Sonde selon l'une des revendications 16 à 20, **caractérisée en ce qu'**il y a dans chaque zone d'émission (1) plusieurs émetteurs (3) alignés selon la direction de propagation.

**22.** Sonde selon l'une des revendications 16 à 21, **caractérisée en ce qu'**avec des émetteurs (3) et récepteurs (4) piézo-électriques chaque zone est encadrée par deux éléments (8) reliés à la masse.

**23.** Sonde selon l'une des revendications 16 à 22, **caractérisée en ce que** chaque émetteur (3) et/ou chaque récepteur (4) est un élément allongé transversalement à la direction de propagation étudiée.

**24.** Sonde selon la revendication 23, **caractérisée en ce que** chaque élément (3,4) présente parallèlement à la direction de propagation une largeur comprise entre environ 100 $\mu$m et environ 1 cm.

**25.** Sonde selon la revendication 23, **caractérisée en ce que** chaque élément (3,4) présente parallèlement à la direction de propagation une largeur à peu près égale à la demi-longueur d'onde dans l'eau pour la fréquence ultra sonore utilisée.

**26.** Sonde selon l'une des revendications 16 à 25, **caractérisée en ce qu'**au moins pour la zone de réception (2), les éléments sont multiples en succession côte à côte.

**27.** Appareil pour évaluer in-vivo et de manière non-invasive les propriétés mécaniques d'un os, **caractérisé en ce qu'**il comprend :

- une sonde (21) selon l'une des revendications 16 à 26,
- des moyens de commande d'émission (26, 46, 38, 41) pour commander de manière décalée dans le temps au moins un signal ultrasonore à partir de chaque zone d'émission (1), dans un sens de propagation respectif vers la zone de réception (2),
- des moyens pour déterminer pour au moins deux récepteurs et pour chaque sens de propagation un écart de temps de parcours respectif ($\Delta t^+$, $\Delta t^+$) jusqu'à ces deux récepteurs déterminés, et
- des moyens(26) pour calculer en fonction des deux écarts de temps de parcours une durée de propagation approximative ($\Delta t_{0, 1,\dots}, \Delta t_k$) comprise entre les deux écarts de temps de parcours ou une vitesse de propagation approximative ($V_{osa0, 1, \dots k}$) correspondante.

**28.** Appareil selon la revendication 27, **caractérisé en ce que** les moyens de calcul (26) mettent en oeuvre les étapes définies à l'une des revendications 3 à 5.

**29.** Appareil selon la revendication 27 ou 28, **caractérisé en ce que** pour chaque sens de propagation, les moyens de commande provoquent l'émission de signaux successifs séparés par une interruption, et il est prévu des moyens d'activation sélective (38, 42) pour activer l'un respectif des au moins deux récepteurs (4) pour chaque signal, et désactiver l'autre, respectivement, des deux récepteurs, et les moyens pour déterminer l'écart de temps sont des moyens pour déterminer la différence entre les temps de parcours des deux signaux, chacun entre la zone d'émission activée (1) et le récepteur (4) respectivement activé.

**30.** Appareil selon l'une des revendications 27 à 29, **caractérisé en ce que** chaque zone d'émission (1) comprend au moins deux émetteurs (3), et les moyens de commande d'émission (26, 46, 38, 41) sont conçus pour activer sélectivement les émetteurs et pour synchroniser en concordance spatiale de phase au moins deux émetteurs (3) situés dans une même zone d'émission (1).

**31.** Appareil selon l'une des revendications 27 à 30, **caractérisé en ce qu'**il comprend un séquenceur (38) qui pilote de manière coordonnée d'une part la sélection des émetteurs (3) et le calage temporel des signaux émis, et d'autre part des moyens collecteurs (37) en ce qui concerne les récepteurs (4) à activer et respectivement à désactiver à la suite de chaque émission.

**Claims**

**1.** Process for evaluating in a non-invasive way the running time and/or the propagation speed of ultrasonic signals between two points along an interface in a body, particularly along a bone surface in which, by means of ultrasound transmitters (3) and receivers (4) placed at the accessible surface of the body along the interface to listen, and distributed along an adapter blade, we determine for each of the two opposed direction of propagation along the interface the running time gap up to at least two receivers spaced along the direction of propagation studied, **characterized in that** we use transmitters (3) placed on either side of the at least two receivers, so that same receptors receive the signals for the two propagation directions.

**2.** Process according to claim 1, **characterized in that** said at least two receivers (4) form a row in the direction (Ox) of propagation and are elongated transversely to the direction of propagation studied, being substantially equally spaced along said direction.

**3.** Process according to claim 1 or 2, **characterized in that** the signal propagation speed along the interface is calculated in an approximate way according to the formula

$$V_{osa0} \cong 2\frac{\Delta r}{\Delta t^+ + \Delta t^-}$$

in which $V_{osaO}$ is the approximate value of the signal propagation speed, $\Delta r$ is the distance between the two receivers, $\Delta t^+$ and $\Delta t^-$ are, for each direction of propagation, respectively, the running time gap of the signal up to the two receivers.

**4.** Process according to claim 1 or 2, **characterized in that** when using a row a receivers (41) in a number greater than 2, the following calculations are proceed with :

- the gradient of a straight line representative of a signal running times depending on the receivers position along the row is determined for each direction of propagation ;
- the signal approximate speed in bone is determined according to the formula :

$$V_{osa0} \cong \frac{2}{\dfrac{1}{V^+} + \dfrac{1}{V^-}}$$

in which

$$\frac{1}{V^+} = \text{gradient of the substantially straight curve of the signal running time depending on the receiver position}$$

in the row in a propagation direction ;

$$\frac{1}{V^-} = \text{gradient of the substantially straight curve of the signal running time depending on the receiver position}$$

in the row in the other propagation direction.

5. Process according to claim 3 or 4, **characterized in that** the accuracy of the signal propagation speed determination is increased by at least one iteration comprising the following three calculations, in which k is the rank of the iteration :

a) evaluation $\beta_k$ of the angle $\beta$ by which the signal cross through the intercalary medium located between the interface and the receivers according to the formula :

$$Sin\beta_k \cong \frac{V_{tme}}{V_{osa(k-1)}}$$

in which :

$V_{tme}$ = signal assumed speed in the intercalary medium ;
$V_{osa(k-1)}$ = last estimated speed of the propagation along the interface.

b) evaluation $\alpha_k$ of the gradient angle ($\alpha$) of the interface with respect to the receivers row according to the angle $\beta_k$ obtained in step (a) :

$$\tan(\alpha_k) = 2\left[\frac{\Delta t^+ - \Delta t^-}{\Delta t^+ + \Delta t^-}\right]\tan\beta_k$$

c) determination of the new approximate value of the propagation speed.

$$V_{osak} = V_{osa0} \, Cos\alpha_k$$

6. Process according to any one of claims 1 to 5, **characterized in that** said running time gap is determined for each propagation direction by doing the difference ($\Delta t^+$, $\Delta t^-$) between the running time of at least a first signal to a first of the at least two receivers, and the running time of at least a second signal to a second of the at least two receivers.

7. Process according to claim 6, **characterized in that** the signals are separated by interruptions.

8. Process according to claim 6 or 7, **characterized in that** only one receiver (4) is activated for each signal.

9. Process according to any one of claims 1 to 8, **characterized in that** receivers (4) used have between them a spacing which is comprised between about 100$\mu$m and about 1 cm.

10. Process according to any one of claims 1 to 9, **characterized in that** signal used have a half-wavelength in water ($\lambda_e/2$) which approximately corresponds to the size of each receiver (41) in the propagation direction studied (Ox).

11. Process according to any one of claims 1 to 10, **characterized in that** at least two transmitters (3) are used for a same propagation direction, which are synchronized to transmit in a spatial phase match.

12. Process according to any one of claims 1 to 11, **characterized in that** receivers (4) used are linked between them in a substantially fixed way in a probe (21).

13. Process according to claim 12, **characterized in that** the probe (21) is calibrated by carrying out a prior measurement of the running time gap of signals up to successive detectors, in both directions, along a reference interface placed parallel to the average direction of alignment of the receivers, then by calculating the distance Δr between two receivers according to the known propagation speed $V_{cal}$ along the interface and time gap $\Delta t^+$ and $\Delta t^-$ in the two propagation direction according to the formula

$$\Delta r = Vcal \ (\Delta t^+ + \Delta t^-)/2$$

14. Process according to any one of claims 1 to 13, **characterized in that** it is applied to the determination of the speed of the first signal received in response to a transmitted signal.

15. Process according to any one of claims 1 to 13, **characterized in that** it is applied to get rid of the temporal deformation due to the intercalary medium from a spatiotemporal image of the received signals by at least two detectors in response to a transmitted signal.

16. Ultrasound probe for bones auscultation for the implementation of a process according to any one of claims 1 to 15, comprising ultrasound transmitters (3) and receivers (4), distributed along an adapter blade, **characterized in that** the transmitters (3) are placed into two transmitting areas (1) located on either side of a receiving area (2) where are the receivers (4), so that the same receivers receive the signals for the two propagation direction.

17. Probe according to claim 16, **characterized by** an absorbing barrier (11) for ultrasound between the receiving area (2) and each of the transmitting areas (1).

18. Probe according to claim 17, **characterized in that** the barrier (11) is in cork.

19. Probe according to claim 17 or 18, **characterized in that** the barrier (11) extends substantially to a face (12) for the contact with the patient's body.

20. Probe according to any one of claims 18 to 20, **characterized in that** the barrier (11) extends towards the rear, protruding in a rear medium (6) forming a support common to the transmitters (3) and the receivers (4).

21. Probe according to any one of claims 16 to 20, **characterized in that** there is in each transmitting areas (1) several transmitters (3) aligned along the propagation direction.

22. Probe according to any one of claims 16 to 21, **characterized in that** with piezo-electrics transmitters (3) and receivers (4), each area is framed by two elements connected to the ground.

23. Probe according to any one of claims 16 to 22, **characterized in that** each transmitters (3) and/or each receivers (4) is an element elongated transversely to the direction of propagation studied.

24. Probe according to claim 23, **characterized in that** each element (3,4) presents in parallel to the propagation direction a width comprised between approximately 100 $\mu$m and approximately 1 cm.

25. Probe according to claim 23, **characterized in that** each element (3,4) presents in the propagation direction a width approximately equal to the half-wavelength in water of the used ultrasonic frequency.

26. Probe according to any one of claims 16 to 25, **characterized in that** at least for the receiving area (2), the elements are multiple in succession side by side.

27. Apparatus for evaluating in vivo and non-invasively the mechanical properties of a bone, **characterized in that** it comprises :

   - probe according to any one of claims 16 to 26,

- emission control means (26, 46, 38, 41) for controlling in a time-staggered way at least one ultrasonic signal from each transmitting area (1), in a respective propagation direction towards the receiving area (2),

- means for determining for at least two receivers and for each propagation direction a respective running time gap ($\Delta t^+$, $\Delta t^-$) to the two determined receivers, and

- means (26) for calculating according to the two running time gaps an approximate propagation time ($\Delta t_{0,\,1},...,$ $\Delta t_k$) comprised between the two running time gaps or a corresponding approximate propagation speed ($\mathbf{V}_{osa0,\,1,\,...k}$).

28. Apparatus according to claim 27, **characterized in that** the calculation means (26) implement the steps defined in one of claims 3 to 5.

29. Apparatus according to claim 27 or 28, **characterized in that** for each propagation direction, the control means cause the emission of successive signals separated by an interruption, and selective activation means (38, 42) are provided to enable the respective one of the at least two receivers (4) for each signal, and to disable the other, respectively, of the two receivers, and the means for determining the time gap are means to determine the gap between the two signals running times, each between the activated transmitting area (1) and the respectively activated receiver (4).

30. Apparatus according to any one of claims 27 to 29, **characterized in that** each transmitting area (1) comprise at least two transmitters (3), and the transmitting control means (26, 46, 38, 41) are designed to enable selectively the transmitters and to synchronize in spatial phase accordance at least two transmitters (3) located in the same transmitting area (1).

31. Apparatus according to any one of claims 27 to 30, **characterized in that** it comprises a sequencer (38) which drives in a coordinated manner on the one hand the transmitters (3) selection and the temporal clamping of transmitted signals, and on the other hand the collecting means (37) with regard to receivers (4) to enable and to respectively turn off following each transmission.

**Patentansprüche**

1. Verfahren zum nicht invasiven Auswerten einer Laufzeit und/oder einer Ausbreitungsgeschwindigkeit von Ultraschallsignalen zwischen zwei Punkten entlang einer Schnittstelle in einem Körper, insbesondere entlang einer Knochenfläche, wobei anhand von Ultraschall-Sendern (3) und -Empfängern (4), die auf der zugänglichen Oberfläche des Körpers entlang der abzuhörenden Schnittstelle angeordnet und entlang einer Adapterlamelle verteilt sind, für jede der beiden entgegengesetzten Ausbreitungsrichtungen entlang der Schnittstelle die Abweichung der Laufzeit bis zu mindestens zwei Empfängern, die in der untersuchten Ausbreitungsrichtung beabstandet sind, bestimmt wird, **dadurch gekennzeichnet, dass** Sender (3) verwendet werden, die auf beiden Seiten der mindestens zwei Empfänger angeordnet sind, so dass die gleichen Empfänger die Signale für die beiden Ausbreitungsrichtungen empfangen.

2. Verfahren nach Anspruch 1, **dadurch gekennzeichnet, dass** die mindestens zwei Empfänger (4) eine Reihe in der Ausbreitungsrichtung (Ox) bilden und sich quer zur untersuchten Ausbreitungsrichtung erstrecken, wobei sie in dieser Richtung im Wesentlichen gleichweit entfernt sind.

3. Verfahren nach Anspruch 1 oder 2, **dadurch gekennzeichnet, dass** die Ausbreitungsgeschwindigkeit des Signals entlang der Schnittstelle gemäß der Formel

$$V_{osa0} \cong 2\frac{\Delta r}{\Delta t^+ + \Delta t^-}$$

approximiert wird, wobei $V_{osa0}$ der approximierte Wert der Ausbreitungsgeschwindigkeit des Signals ist, $\Delta r$ der Abstand zwischen den beiden Empfängern, $\Delta t^+$ und $\Delta t^-$ jeweils für jede Ausbreitungsrichtung die Laufzeitabweichung des Signals bis zu den beiden Empfängern ist.

4. Verfahren nach Anspruch 1 oder 2, **dadurch gekennzeichnet, dass**, wenn man eine Reihe mit mehr als 2 Empfängern (41) verwendet, folgende Berechnungen vorgenommen werden:

- für jede Ausbreitungsrichtung Bestimmen der Neigung einer Geraden, welche die Laufzeiten eines Signals in Abhängigkeit von der Position der Empfänger entlang der Reihe darstellt;
- Bestimmen der approximierten Geschwindigkeit des Signals im Knochen gemäß der Formel

$$V_{osa0} \cong \frac{2}{\dfrac{1}{V^+} + \dfrac{1}{V^-}}$$

wobei

$$\frac{1}{V^+} = \texttt{Neigung}$$ Neigung der im Wesentlichen geradlinigen Kurve der Laufzeit des Signals in Abhängigkeit von der Position des Empfängers in der Reihe in einer Ausbreitungsrichtung ist;

$$\frac{1}{V^-} = \texttt{Neigung}$$ der im Wesentlichen geradlinigen Kurve der Laufzeit des Signals in Abhängigkeit von der Position des Empfängers in der Reihe in der anderen Ausbreitungsrichtung ist.

**5.** Verfahren nach Anspruch 3 oder 4, **dadurch gekennzeichnet, dass** die Präzision der Bestimmung der Ausbreitungsgeschwindigkeit des Signals durch mindestens eine Iteration zunimmt, welche die drei folgenden Berechnungen umfasst, bei denen k den Rang der Iteration darstellt:

a) Auswerten $\beta_k$ des Winkels $\beta$ je nachdem, ob das Signal durch das Zwischenmedium geht, das sich zwischen der Schnittstelle und den Empfängern befindet, durch die Formel:

$$Sin\beta_k \cong \frac{V_{tme}}{V_{osa(k-1)}}$$

wobei:

$V_{tme}$ = angenommene Geschwindigkeit des Signals im Zwischenmedium;
$V_{osa(k-1)}$ = letzte geschätzte Geschwindigkeit der Ausbreitung entlang der Schnittstelle.

b) Auswerten $\alpha_k$ des Neigungswinkels ($\alpha$) der Schnittstelle im Verhältnis zu der Reihe von Empfängern in Abhängigkeit von dem in Schritt (a) erzielten Winkel $\beta_k$:

$$\tan(\alpha_k) = 2\left[\frac{\Delta t^+ - \Delta t^-}{\Delta t^+ + \Delta t^-}\right] \tan\beta_k$$

c) Bestimmen des neuen approximierten Wertes der Ausbreitungsgeschwindigkeit

$$V_{osak} = V_{osa0}\, Cos\alpha_k.$$

**6.** Verfahren nach einem der Ansprüche 1 bis 5, **dadurch gekennzeichnet, dass** für jede Ausbreitungsrichtung die Laufzeitabweichung bestimmt wird, indem die Differenz ($\Delta t^+$, $\Delta t^-$) zwischen der Laufzeit mindestens eines ersten Signals bis zu einem ersten der mindestens zwei Empfänger und der Laufzeit mindestens eines zweiten Signals bis zu einem zweiten der mindestens zwei Empfänger gebildet wird.

**7.** Verfahren nach Anspruch 6, **dadurch gekennzeichnet, dass** die Signale durch Unterbrechungen getrennt sind.

**8.** Verfahren nach Anspruch 6 oder 7, **dadurch gekennzeichnet, dass** für jedes Signal ein einziger Empfänger (4) aktiviert wird.

**9.** Verfahren nach einem der Ansprüche 1 bis 8, **dadurch gekennzeichnet, dass** man Empfänger (4) verwendet, die zueinander eine Beabstandung aufweisen, die zwischen ungefähr 100 $\mu$m und ungefähr 1 cm liegt.

**10.** Verfahren nach einem der Ansprüche 1 bis 9, **dadurch gekennzeichnet, dass** ein Signal verwendet wird, dessen Halbwellenlänge im Wasser ($\lambda_e$/2) ungefähr der Dimension jedes Empfängers (41) in der untersuchten Ausbreitungsrichtung (Ox) entspricht.

**11.** Verfahren nach einem der Ansprüche 1 bis 10, **dadurch gekennzeichnet, dass** für ein und dieselbe Ausbreitungsrichtung mindestens zwei Sender (3) verwendet werden, die synchronisiert werden, um räumlich phasengleich zu senden.

**12.** Verfahren nach einem der Ansprüche 1 bis 11, **dadurch gekennzeichnet, dass** Empfänger (4) verwendet werden, die in einer Sonde (21) im Wesentlichen fest miteinander verbunden sind.

**13.** Verfahren nach Anspruch 12, **dadurch gekennzeichnet, dass** die Sonde (21) kalibriert wird, indem eine vorbereitende Messung der Abweichung der Signallaufzeit bis zu den aufeinander folgenden Detektoren in beiden Richtungen entlang einer Referenzschnittstelle vorgenommen wird, die parallel zur mittleren Ausrichtungsrichtung der Empfänger angeordnet ist, und dann der Abstand $\Delta$r zwischen zwei Empfängern in Abhängigkeit von der bekannten Ausbreitungsgeschwindigkeit $V_{cal}$ entlang der Schnittstelle und der Zeitabweichungen $\Delta t^+$ und $\Delta t^-$ in den beiden Ausbreitungsrichtungen durch die Formel $\Delta r = Vcal\ (\Delta t^+ + \Delta t^-)/2$ berechnet wird.

**14.** Verfahren nach einem der Ansprüche 1 bis 13, **dadurch gekennzeichnet, dass** es auf die Bestimmung der Geschwindigkeit des ersten Signals, das als Antwort auf ein gesendetes Signal empfangen wird, angewendet wird.

**15.** Verfahren nach einem der Ansprüche 1 bis 13, **dadurch gekennzeichnet, dass** es angewendet wird, um ein räumlich und zeitliches Bild der Signale, die von mindestens zwei Detektoren als Antwort auf ein gesendetes Signal empfangen werden, von der zeitlichen Verformung zu befreien, die auf das Zwischenmedium zurückzuführen ist.

**16.** Ultraschallsonde zur Knochenuntersuchung zum Umsetzen eines Verfahrens nach einem der Ansprüche 1 bis 15, umfassend Ultraschall-Sender (3) und -Empfänger (4), die an einer Adapterlamelle entlang verteilt sind, **dadurch gekennzeichnet, dass** die Sender (3) in zwei Sendezonen (1) angeordnet sind, die auf beiden Seiten einer Empfangszone (2) liegen, in der sich die Empfänger (4) befinden, so dass die gleichen Empfänger die Signale für die beiden Ausbreitungsrichtungen empfangen.

**17.** Sonde nach Anspruch 16, **gekennzeichnet durch** eine Schranke (11), die für den Ultraschall zwischen der Empfangszone (2) und jeder der Sendezonen (1) absorbierend ist.

**18.** Sonde nach Anspruch 17, **dadurch gekennzeichnet, dass** die Schranke (11) aus Kork besteht.

**19.** Sonde nach Anspruch 17 oder 18, **dadurch gekennzeichnet, dass** sich die Schranke (11) im Wesentlichen für den Kontakt mit dem Körper des Patienten bis zu einer Seite (12) erstreckt.

**20.** Sonde nach einem der Ansprüche 18 bis 20, **dadurch gekennzeichnet, dass** sich die Schranke (11) nach hinten erstreckt, wobei sie in ein rückseitiges Medium (6) vorsteht, das eine Auflage bildet, die den Sendern (3) und den Empfängern (4) gemeinsam ist.

**21.** Sonde nach einem der Ansprüche 16 bis 20, **dadurch gekennzeichnet, dass** es in jeder Sendezone (1) mehrere Sender (3) gibt, die in der Ausbreitungsrichtung ausgerichtet sind.

**22.** Sonde nach einem der Ansprüche 16 bis 21, **dadurch gekennzeichnet, dass** mit piezoelektrischen Sendern (3) und Empfängern (4) jede Zone von zwei Elementen (8) eingerahmt ist, die an Masse gelegt sind.

**23.** Sonde nach einem der Ansprüche 16 bis 22, **dadurch gekennzeichnet, dass** jeder Sender (3) und/oder jeder Empfänger (4) ein Element ist, das sich quer zur untersuchten Ausbreitungsrichtung erstreckt.

**24.** Sonde nach Anspruch 23, **dadurch gekennzeichnet, dass** jedes Element (3, 4) parallel zur Ausbreitungsrichtung eine Breite aufweist, die zwischen ungefähr 100 μm und ungefähr 1 cm liegt.

**25.** Sonde nach Anspruch 23, **dadurch gekennzeichnet, dass** jedes Element (3, 4) parallel zur Ausbreitungsrichtung eine Breite aufweist, die in etwa gleich der Halbwellenlänge im Wasser für die verwendete Ultraschallfrequenz ist.

**26.** Sonde nach einem der Ansprüche 16 bis 25, **dadurch gekennzeichnet, dass** die Elemente mindestens für die Empfangszone (2) der Reihe nach nebeneinander in mehreren Exemplaren vorliegen.

**27.** Gerät zum nicht invasiven in-vivo-Auswerten der mechanischen Eigenschaften eines Knochens, **dadurch gekennzeichnet, dass** es Folgendes umfasst:

- eine Sonde (21) nach einem der Ansprüche 16 bis 26,
- Sendesteuermittel (26, 46, 38, 41) zum zeitversetzten Steuern mindestens eines Ultraschallsignals von jeder Sendezone (1) aus in einer jeweiligen Ausbreitungsrichtung zur Empfangszone (2),
- Mittel zum Bestimmen für mindestens zwei Empfänger und für jede Ausbreitungsrichtung einer jeweiligen Laufzeitabweichung ($\Delta t^+$, $\Delta t^-$) bis zu diesen beiden bestimmten Empfängern, und
- Mittel (26) zum Berechnen in Abhängigkeit von den beiden Laufzeitabweichungen einer ungefähren Ausbreitungsdauer ($\Delta t_{0, 1}, ..., \Delta t_k$), die zwischen den beiden Laufzeitabweichungen liegt, oder einer entsprechenden ungefähren Ausbreitungsgeschwindigkeit ($V_{osao, 1, ... k}$).

**28.** Gerät nach Anspruch 27, **dadurch gekennzeichnet, dass** die Rechenmittel (26) die Schritte umsetzen, die in einem der Ansprüche 3 bis 5 definiert sind.

**29.** Gerät nach Anspruch 27 oder 28, **dadurch gekennzeichnet, dass** für jede Ausbreitungsrichtung die Steuermittel die Sendung aufeinander folgender Signale verursachen, die durch eine Unterbrechung getrennt sind, und Mittel zum selektiven Aktivieren (38, 42) bereitgestellt werden, um einen jeweiligen der mindestens zwei Empfänger (4) für jedes Signal zu aktivieren, und jeweils den anderen der beiden Empfänger zu deaktivieren, und die Mittel zum Bestimmen der Zeitabweichung Mittel sind, um den Unterschied zwischen den Laufzeiten der beiden Signale zu bestimmen, und zwar jeweils zwischen der aktivierten Sendezone (1) und dem jeweils aktivierten Empfänger (4).

**30.** Gerät nach einem der Ansprüche 27 bis 29, **dadurch gekennzeichnet, dass** jede Sendezone (1) mindestens zwei Sender (3) umfasst, und die Sendesteuermittel (26, 46, 38, 41) ausgelegt sind, um selektiv die Sender zu aktivieren und um mindestens zwei Sender (3), die in einer selben Sendezone (1) liegen, räumlich phasengleich zu synchronisieren.

**31.** Gerät nach einem der Ansprüche 27 bis 30, **dadurch gekennzeichnet, dass** es einen Folgeschalter (38) umfasst, der einerseits die Auswahl der Sender (3) und die zeitliche Einstellung der gesendeten Signale und andererseits Sammelmittel (37) bezüglich der Empfänger (4), die jeweils nach jeder Sendung zu aktivieren und deaktivieren sind, koordiniert ansteuert.

FIG.1

FIG.2

# FIG.3

Module électronique Emetteur/Recepteur

# FIG.4

49

j de 1 à r

Tir du groupe
d'émetteurs

n tirs
sur $r_j$

Délai
> 125µs

réception
$r_j$

Stockage temporaire
du tir courant sur $r_j$

Stockage en mémoire de
la moyenne des n signaux
reçus par $r_j$

## FIG.5

## FIG.6

exemple pour $\alpha = 4°$

pente : $1/V_-$

pente : $1/V_+$

$V_+ \neq V_-$

t µs

position du récepteur r (mm)

r (mm)

$R_1$   $R_2$       $R_r$   $\alpha = 4°$

sens +        •52        4        sens –

# FIG_7

alignement moyen
de la face avant de
la sonde

$\Delta r_1$?   $R_2$          $R_4$      $R_5$        $R_6$

$R_1$    $\Delta r_2$?   $R_3$                    $\Delta r_5$?

Tissus mous

Os

# FIG_8

temps

Droite de régression
de pente $1/V_{Os}$

$\Delta t_2$

$\Delta t_1$   $\Delta r_2$

$\Delta r_1$

distance

FIG_9

FIG.10

(a) Plexiglas®/Eau

(b) Aluminium/Eau

FIG.11

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 9526160 A
- SU 1175435
- SU 1308319
- SU 1342479
- US 5143072 A
- US 5143069 A
- WO 9713145 A
- WO 9945348 A
- SU 1420383
- US 5426979 A
- WO 0024307 A

**Littérature non-brevet citée dans la description**

- **G. LOWET ; G. VAN DER PERRE.** Ultrasound velocity measurement in long bones: Measurement method and simulation of ultrasound wave propagation. *J Biomech,* 1996, vol. 29 (10), 1255-1262
- **I. SIEGEL ; G. T. ANAST ; T. MELDS.** The determination of fracture healing by measurement of sound velocity across the fracture site. *Surgery, Gynecol, Obstetrics,* 1958, 327-332
- **C. NJEH ; D. HANS ; T. FUERST ; C. C. GLÜER ; H. K. GENANT.** *Quantitative ultrasound assessment of osteoporosis and bone status. London,* 1999
- **M. WEISS ; A. BEN-SHLOMO ; P. HAGAG ; S. ISH-SHALOM.** Discrimination of proximal hip fracture by quantitative ultrasound measurement at the radius. *Osteoporosis Int.,* 2000, vol. 11, 411-416
- **J.L MARI.** Géophysique de gisement et de génie civil. Publications de l'institut Français du Pétrole
- **M. WEISS ; A. BEN-SHLOMO ; P. HAGAG ; S. ISH-SHALOM.** Discrimination of proximal hip fracture by quantitative ultrasound measurement at the radius. *Osteoporosis Int. 11,* 2000, 411-416
- **E. BOSSY ; M. TALMANT ; P.LAUGIER.** Axial transmission of 1 MHz ultrasonic waves on thin cortical bone plates: a simulation study. *IEEE UFFC 2001 International Conférence*
- **R. BARKMANN ; E. KANTOROVICH ; C. SINGAL ; D. HANS ; H.K. GENANT ; M. HELLER ; C.C. GLÜER.** A new method for quantitative ultrasond measurements at multiple skeletal sites. *Journal of Clinical Densitometry,* 2000, vol. 3, 1-7
- **M. R. STEGMAN ; R.P. HEANEY ; D. TRAVERS-GUSTAFSON ; J. LEIST.** Cortical ultrasound velocity as an indicator of bone status. *Osteoporosis International,* 1995, vol. 5, 349-353
- **D.L. FOLDS.** Expérimental détermination of ultrasonic wave velocities in plastics, elastomers, and synthetic foam as a function of temperature. *J. Acoust. Soc. Am.,* 1972, vol. 52, 426-427
- **E. BOSSY ; M. TALMANT ; P. LAUGIER.** Effect of bone cortical thickness on velocity measurements using ultrasonic axial transmission : a 2D simulation study. *J. Acoust. -Soc. Am.,* 2002, vol. 112, 297-307